# EUROPÄISCHE PATENTSCHRIFT

(11) **EP 3 075 357 B1**
(45) Veröffentlichungstag und Bekanntmachung des Hinweises auf die Patenterteilung: **15.08.2018**
(21) Anmeldenummer: 16158937.9
(22) Anmeldetag: 07.03.2016
(51) Int. Cl.: A61F 2/30, A61F 2/36

(54) **SPACERFORM UND VERFAHREN ZUR HERSTELLUNG VON HÜFTSPACERN**
SPACER MOULD AND METHOD FOR PRODUCING HIP SPACERS
MOULE D'ECARTEUR ET PROCEDE DE FABRICATION D'ECARTEUR COXAUX

(30) Priorität: 27.03.2015 DE 102015104704
(43) Veröffentlichungstag der Anmeldung: 05.10.2016
(73) Patentinhaber: Heraeus Medical GmbH, 61273 Wehrheim (DE)
(72) Erfinder: Wüst, Edgar, 34 Groß-Umstadt (DE); Dr. Vogt, Sebastian, 99092 Erfurt (DE)
(74) Vertreter: Heraeus IP

(56) Entgegenhaltungen:
- EP-A1- 2 522 310
- EP-A1- 2 787 928
- CN-U- 203 619 725
- DE-U1-202009 012 964

## Beschreibung

Die Erfindung betrifft eine Spacerform zur Herstellung eines temporären Hüftspacers für eine zweizeitige Revision. Die Erfindung betrifft auch ein Verfahren zur Herstellung eines solchen Hüftspacers.

Zementierte und nichtzementierte Totalgelenkendoprothesen (TEP) sind heute Stand der Technik in der Orthopädie. Leider sind gibt es eine geringe Anzahl, ca. 2-6 %, von Früh- und Spätinfektionen bei TEPs. Infizierte TEPs machen im Allgemeinen eine Wechsel-Operation notwendig. Diese Wechsel-Operationen werden in einzeitige und zweizeitige Operationen unterteilt. Bei der zweizeitigen Wechsel-Operation wird zuerst die infizierte TEP entfernt, das infizierte Knochen- und Weichgewebe debridiert und anschließend ein Spacer als temporärer Platzhalter eingesetzt. Dabei kommen üblicherweise industriell vorgefertigte Spacer zur Anwendung, die ein Antibiotikum enthalten können, oder auch individuelle aus Polymethylmethacrylat-Knochenzement (PMMA-Knochenzement) gefertigte Spacer, die entweder frei geformt, oder auch unter Verwendung von Gießformen vom Operateur selbst gefertigt werden. Diese Spacer können durch individuelle Dotierung des eingesetzten PMMA-Knochenzements mit Antibiotika, entsprechend dem Antibiogramm der der Infektion zu Grunde liegenden Keime patientenspezifisch gefertigt werden.

Die gegenwärtig kommerziell verfügbaren, industriell vorgefertigten Spacer gibt es in verschieden Größen und Geometrien, die dem größten Teil der möglichen anatomischen Situationen mehr oder weniger gut entsprechen. Solche vorgefertigten Spacer für Hüftgelenke (Hüftspacer) enthalten einen Stahlkern, um eine ausreichende mechanische Stabilität des Hüftspacers zu gewährleisten.

Neben der freien Formgebung von Spacern durch Operateure werden auch Gießformen verwendet. In diese Gießformen wird normalerweise durch Einfüllöffnungen PMMA-Zementteig mit Hilfe eines Zementiersystems und unter Verwendung von manuell angetriebenen Austragsvorrichtungen eingepresst. Die Gießformen müssen deshalb dem Einpressdruck standhalten, ohne dass die Gießformen auseinander getrieben werden oder sich verformen. Geeignete Verschlüsse zum Fixieren der Gießformen sind daher notwendig.

Spacer verbleiben meistens mehrere Wochen, vielfach 6 bis 8 Wochen, im Patienten, bis sich die Infektion beruhigt hat. Anschließend wird in einer zweiten Operation der Spacer entfernt, das angrenzende Gewebe nochmals debridiert und dann wird die Revisionsprothese eingesetzt, die zementiert oder auch zementfrei eingesetzt werden kann.

Eine einfache, zweiteilige, aus transluzentem Kunststoff gefertigte Gießform für Hüftspacer wird in dem Patent US 6 361 731 B1 offenbart. Bei dieser Vorrichtung gibt es einen Port als Eifüllöffnung, in den der PMMA-Knochenzementteig mit Hilfe eines Austragsrohrs eines Zementiersystems eingespritzt wird. Eine Befüllung dieser Gießform ist nur mit einem bei Raumtemperatur niedrigviskosem PMMA-Knochenzementteig möglich. Dazu wird entweder niedrigviskoser PMMA-Knochenzement eingesetzt oder es wird ein bei Raumtemperatur hochviskoser PMMA-Knochenzement verwendet, der jedoch durch Vorkühlung der Zementkomponenten unmittelbar nach Vermischung für einige Minuten eine geringere Viskosität besitzt, die vergleichbar zu dem bei Raumtemperatur niedrigviskosen PMMA-Knochenzement gießfähig ist.

In der US 2007/0 222 114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerformsegmente werden mittels Schraubschellen aneinandergefügt. Durch Kanäle in der Spacerform wird der PMMA-Knochenzementteig eingeführt. Durch den komplexen Aufbau der Gießform ist es sehr aufwändig, die Spacerformsegmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Hüftschafts anpassen zu können. Der Teil, der den Hüftschaft bildet, besteht aus zwei spiegelbildlichen Halbschalen, die auseinandergenommen werden können. Der PMMA-Knochenzementteig wird über eine Zuführöffnung in den Hohlraum eingeführt. Die einzelnen Formteile sind mit Hilfe von Schraubverbindungen miteinander verbunden. Zur Entnahme des Spacers müssen sämtliche Schrauben gelöst werden. Damit erfordert die intraoperative Herstellung relativ viel Zeit mit dem Risiko einer erhöhten Infektionsrate. Die Kleinteile erschweren die Handhabung im oft hektischen Operationsalltag.

Eine weitere Gießform ist in der EP 2 522 310A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers.

In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente.

Zur EP 2 787 928 A1 ähnliche Gießformen wurden im Patent US 7 789 646 B2 und im Patent US 8 801 983 B1 beschrieben.

In DE 20 2009 012964 U1 wird eine mehrteilige Gießform beschrieben, die die Herstellung einer temporären Gelenkprothese ermöglicht, welche nur einen Gelenkkopf umfasst. Der Gelenkkopf umfasst eine eingeformte Konusaufnahme, so dass der Gelenkkopf auf einen Konus des Schaftabschnitts einer Gelenkprothese, die im Knochen implantiert ist, gesteckt werden kann. Die bekannten Spacerformen und Verfahren zur Herstellung von Hüftspacern haben den Nachteil, dass die Spacerform als Gießform aufwendig und mit hohem Druck mit Knochenzement gefüllt werden muss. Entweder muss ein relativ dünnflüssiger Knochenzement durch Einfüllöffnungen eingespeist werden oder eine flexible Spacerform muss sich beim Aufnehmen des Knochenzements ausdehnen. Durch die dabei auftretenden hohen Kräfte müssen die Teile solcher Spacerformen meist aufwendig miteinander verbunden und gegeneinander stabilisiert werden. Die hierfür notwendigen Teile sind komplex und damit teuer im Aufbau und/oder vielteilig, was beim Zusammensetzen im oft hektischen OP-Alltag nachteilig ist.

Die Aufgabe der Erfindung besteht also darin, die Nachteile des Stands der Technik zu überwinden. Insbesondere soll es die zu entwickelnde Vorrichtung dem medizinischen Personal erlauben, unter OP-Bedingungen mit antibiotisch dotiertem PMMA-Knochenzementteig einen Spacer zu erzeugen, dessen Form den individuellen anatomischen Gegebenheiten des Patienten nahe kommt.

Die zu entwickelnde Spacerform soll so beschaffen sein, dass eine Herstellung von Hüftspacern mit PMMA-Knochenzement unter Verwendung von Vakuummischsystemen und auch ohne Verwendung dieser Mischsysteme, beispielsweise unter Nutzung von einfachen Mischbechern und Spateln, möglich ist. Mit der zu entwickelnden Spacerform sollen Hüftspacer sowohl mit bei Raumtemperatur hochviskosen als auch mit niedrigviskosen PMMA-Knochenzementen herstellbar sein. Die Vorrichtung soll die Ausbildung von scharfen Graten am Spacer möglichst weitgehend konstruktiv ausschließen, da diese vor der Implantation aufwendig mechanisch entfernt werden müssen. Die Vorrichtung soll ohne Ausübung von hohen Drucken, wie sie bei bisher bekannten Vorrichtungen bei der Befüllung mit Vakuummischsystemen auftreten, mit PMMA-Knochenzementteig befüllt werden können. Dadurch und im Allgemeinen sollen möglichst keine separaten Zuhaltungselemente, wie Clips oder Schrauben notwendig sein. Die Spacerform soll des Weiteren möglichst keine separaten, scharfkantigen Verriegelungselemente zum Fixieren von Elementen der Vorrichtung enthalten. Die Spacerform soll aus hygienischen Gründen als Wegwerfprodukt für den einmaligen Gebrauch geeignet sein und dafür möglichst kostengünstig in der Herstellung sein.

Die Aufgaben der Erfindung werden gelöst durch eine Spacerform zur Herstellung eines Hüftspacers, wobei die Spacerform die folgenden Teile aufweist:
A) ein Basiselement, in dem eine Hohlform als Negativ einer Seite eines Femurschafts des zu erzeugenden Spacers vorgesehen ist, wobei am proximalen Ende der Hohlform im Basiselement eine Ausnehmung angeordnet ist, die mit der Hohlform verbunden ist,
B) einen Halbkugeleinsatz, der eine Femurkopfform als Negativ einer Seite eines Femurkopfs des zu erzeugenden Spacers aufweist, wobei der Halbkugeleinsatz in der Ausnehmung im Basiselement an einer der Verbindung zur Hohlform gegenüberliegenden Seite der Ausnehmung angeordnet ist oder anzuordnen ist, so dass die Femurkopfform in Richtung zur Hohlform ausgerichtet ist,
C) einen Adaptereinsatz, der als zweiseitig offener Hohlkörper ausgebildet ist und der in der Ausnehmung des Basiselements zwischen dem Halbkugeleinsatz und der Hohlform angeordnet ist oder anzuordnen ist, so dass die Ausnehmung einen Druck auf den Adaptereinsatz und den Halbkugeleinsatz ausübt, wenn der Adaptereinsatz und der Halbkugeleinsatz in die Ausnehmung eingesetzt sind, wobei der Hohlkörper des Adaptereinsatzes die Femurkopfform des Halbkugeleinsatzes mit der Hohlform im Basiselement verbindet und
D) einen Stempel, der eine Stempelhohlform in Form des Negativs des restlichen Femurschafts aufweist und der auf der Oberseite des Basiselements angeordnet ist oder aufzudrücken ist,
wobei am Rand der Hohlform des Basiselements und/oder am Rand der Stempelhohlform des Stempels eine die Verbindung der Hohlform des Basiselements zur Stempelhohlform des Stempels begrenzende Schneidkante angeordnet ist und am Rand der Femurkopfform und/oder an dem Rand der Öffnung des Hohlkörpers des Adaptereinsatzes zur Femurkopfform eine die Verbindung der Femurkopfform zu dieser Öffnung des Hohlkörpers des Adaptereinsatzes begrenzende Schneidkante angeordnet ist.

Eine erfindungsgemäße Spacerform muss zur Herstellung eines Hüftspacers (im Folgenden auch teilweise nur Spacer genannt) mit einem Kopf und einem Femurschaft geeignet sein. Die Spacerform muss dazu im bestimmungsgemäß zusammengesetzten Zustand ein Negativ des Kopfs des Hüftspacers und ein Negativ des Femurschafts des Hüftspacers bilden. Die beiden Negative (des Kopfs und des Schafts) müssen miteinander verbunden sein, also eine gemeinsame Form als Negativ des Hüftspacers bilden. Dazu sind die Form des Kopfs und die Form des Femurschafts über eine Form eines Halses miteinander verbunden, die ein Negativ des Halses des zu erzeugenden Hüftspacers bildet.

Unter einem Halbkugeleinsatz ist nicht eine im streng geometrischen Sinn geformte Halbkugel als Femurkopfform zu verstehen. Der Halbkugeleinsatz kann ein Negativ eines Kugelabschnitts oder eines Abschnitts einer groben Kugelform formen, der zur Ausformung des Kopfs des Hüftspacers geeignet ist.

Das proximale Ende eines Femurschafts ist das Ende, an dem über den Hals der Kopf des Femur mit dem Femurschaft verbunden ist. Der Kopf des Femur, der eine annähernd kugelförmige Gelenkfläche aufweist, bildet eine Verbindung (Gleitfläche) mit den Beckenknochen und somit das Hüftgelenk. Für den Hüftspacer und auch für die Spacerform, die ja ein Negativ des zu erzeugenden Hüftspacers bildet, werden vorliegend die gleichen anatomischen Bezeichnungen verwendet. Beim Hüftspacer ist der Femurschaft selbstverständlich kürzer gehalten als bei einem echten Femur, da ja nicht der gesamte Femur ersetzt werden soll, sondern vor allem der Kopf mit dessen Gleitfläche. Auch ist der Femurschaft des Hüftspacers meist schmaler beziehungsweise schlanker gestaltet, um den Hüftspacer mit dem Femurschaft im Femur (beziehungsweise im Schaft des Femur) des Patienten verankern zu können.

Die Seite des Femurschafts, die als Negativ durch die Hohlform des Basiselements und durch die Stempelhohlform des Stempels nachgebildet ist, ist in axialer Richtung des Schafts ausgerichtet. Die axiale Richtung des Schafts, beziehungsweise des nachgebildeten Teils des Femurs, ist vorzugsweise parallel zur offenen Oberseite der Hohlform ausgerichtet, besonders bevorzugt liegt die Achse des Femurschafts in der offenen Fläche der Hohlform. Die offene Oberseite der Hohlform ist durch den Rand begrenzt. Alternativ kann die axiale Richtung auch einen Winkel von maximal 30° zur der offenen Fläche der offenen Hohlform einnehmen, bevorzugt einen Winkel von maximal 10° zur der offenen Fläche der offenen Hohlform einnehmen. Das gleiche gilt entsprechend für die Stempelhohlform.

Der restliche Femurschaft beziehungsweise das Negativ des restlichen Femurschafts im Sinne der vorliegenden Erfindung ist die Oberfläche des Negativs des Femurschafts des zu erzeugenden Hüftspacers, die weder von der Hohlform des Basiselements noch von dem Inneren des Hohlkörpers des Adaptereinsatzes nachgebildet wird. Die Stempelhohlform des Stempels erzeugt also die restliche Oberfläche des Femurschafts beziehungsweise des Hüftspacers durch Aufdrücken des Stempels auf die im Überschuss mit PMMA-Knochenzement gefüllten restlichen zusammengesetzten Formteile (Hohlform, Femurkopfform und Hohlkörper).

Wenn im Rahmen der vorliegenden Erfindung von Schneidkanten (im Plural) die Rede ist so ist damit immer auch eine umlaufende einteilige Schneidkante gemeint, die als einzelne Schneidkante angesehen werden könnte. Eine Schneidkante hat im Querschnitt einen Winkel (auch Keilwinkel genannt) von weniger als 90°, bevorzugt von maximal 45°. Eine Schneidkante kann auch als Schneide bezeichnet werden. Die Schneidkanten können nur auf einer Seite angeordnet sein oder auf beiden Seiten der zu verbindenden Formteile. Nur in der Verbindung vom Stempel zum Adaptereinsatz ist bevorzugt keine Schneidkante vorgesehen, da beim Stempeln in der Richtung des Basiselements kein Schneiden erfolgen kann.

Es kann erfindungsgemäß auch vorgesehen sein, dass das Basiselement neben der Ausnehmung in Bezug auf die Ausnehmung nach außen abfällt.

Erfindungsgemäß bevorzugt ist der Stempel auf dem Basiselement derart anzulegen, dass kein durchgehender Spalt zwischen dem Basiselement und dem Stempel verbleibt, insbesondere kein durchgehender Spalt zwischen dem Rand der Hohlform des Basiselements und dem Stempel verbleibt. Bevorzugt können die Hohlform, die Femurkopfform, die Stempelhohlform und der Hohlkörper derart aneinander angelegt und/oder miteinander verbunden werden, dass eine geschlossene innere Form für den Hüftspacer gebildet wird, wobei Entlüftungsöffnungen zur Entlüftung der inneren Form (der Spacerform) vorgesehen sein können.

Mit der vorliegenden Erfindung wird auch vorgeschlagen, dass die Femurkopfform einen umlaufenden Rand aufweist und die Oberflächenkontur des umlaufenden Rands der Femurkopfform des Halbkugeleinsatzes ausgehend von der Femurkopfform nach außen abfallend ist.

Nach außen abfallend ist hier in Bezug auf die Ebene des Rands der Femurkopfform gemeint. Hierdurch kann sichergestellt werden, dass abgeschnittene PMMA-Knochenzementreste von der Femurkopfform entfernt werden beziehungsweise abfallen und zwar in den breiter werdenden Spalt zwischen dem Halbkugeleinsatz und dem Adaptereinsatz.

Ferner kann erfindungsgemäße vorgesehen sein, dass die Schneidkanten die gesamte Hohlform im Basiselement begrenzen und/oder die Schneidkanten die gesamte Femurkopfform im Halbkugeleinsatz begrenzen und/oder der gesamte Hohlkörper des Adaptereinsatzes in der Verbindung zur Femurkopfform durch eine Schneidkante begrenzt ist.

Hierdurch wird sichergestellt, dass der zu erzeugende Hüftspacer durch die Schneidkanten vollständig entgratet wird. Es kann auch vorgesehen sein, dass die Schneidkanten die gesamte Hohlform im Basiselement außer in der Verbindung zur Ausnehmung begrenzen.

Bei einer besonders bevorzugten Weiterbildung der vorliegenden Erfindung kann vorgesehen sein, dass das Basiselement neben der Hohlform in Bezug auf einen Rand der Hohlform nach außen abfällt.

Dass das Basiselement neben der Hohlform in Bezug auf einen Rand der Hohlform nach außen abfällt, bedeutet, dass wenn das Basiselement eine ebene Unterseite hat, beispielsweise zum Aufstellen des Basiselements auf einer ebenen Fläche wie einem Tisch, dass dann die Dicke des Basiselements senkrecht zur ebenen Unterseite vom Rand der Hohlform ausgehend nach außen abnimmt. Die Hohlform ist dabei auf der dieser ebenen Unterseite gegenüberliegenden Oberseite des Basiselements angeordnet. Der Rand der Hohlform fehlt dabei in der Verbindung der Hohlform zur Ausnehmung, das heißt, dass die Hohlform an der Verbindung zur Ausnehmung keinen Rand im Sinne der vorliegenden Erfindung aufweist. Dennoch können an der Verbindung der Hohlform zur Ausnehmung Schneidkanten angeordnet sein, mit denen überschüssiger PMMA-Knochenzement abgetrennt werden kann, der in der Verbindung von der Hohlform des Basiselements zum Hohlkörper des Adaptereinsatzes aus der Spacerform austritt. Wenn das Basiselement keine ebene Unterseite aufweist, beispielsweise weil das Basiselement eine andere Aufstellmöglichkeit hat, dann lässt sich dennoch eine Ebene parallel zum Rand der Hohlform definieren, so dass der Abstand der Oberseite des Basiselements zu dieser gedachten Ebene (beispielsweise parallel zum Boden des Aufstellorts oder zu einer Tischplatte) ausgehend vom Rand der Hohlform nach außen abnimmt. Der Neigungswinkel am Rand der Hohlform beträgt bevorzugt 1° bis 60°, besonders bevorzugt 1° bis 20°. Für den Rand der Femurkopfform kann diese Betrachtung analog durchgeführt werden und es können die gleichen Neigungswinkel vorgesehen sein.

Durch die Neigung kann abgetrennter PMMA-Knochenzement nach außen abfallen beziehungsweise nach außen abrutschen. Dadurch wird vermieden, dass der aushärtende PMMA-Knochenzement, die Teile des Spacerform miteinander verbindet.

Erfindungsgemäße Spacerformen können sich auch dadurch auszeichnen, dass das Basiselement an der Unterseite eine ebene Auflagefläche oder einen Standfuß zum Aufstellen der Spacerform auf einer ebenen Unterlage aufweist.

Hierdurch wird die Anwendung der Spacerform vereinfacht. Die Spacerform kann einfach auf einem Tisch aufgestellt werden, um den Halbkugeleinsatz und den Adaptereinsatz einzustecken und um die Hohlform von oben mit PMMA-Knochenzement zu befüllen. Vorliegend werden die Begriffe oben und unten immer in Bezug auf eine derartige Aufstellung des Basiselements verwendet.

Des Weiteren kann vorgesehen sein, dass der Adaptereinsatz ein zweiteiliger oder mehrteiliger Adaptereinsatz ist, wobei die Teile des Adaptereinsatzes aneinander zu befestigen oder aneinander anlegbar sind, wobei bevorzugt an den Teilen des Adaptereinsatzes Schneidkanten vorgesehen sind, wobei die Schneidkanten an den Rändern der den Hohlkörper bildenden Formen, die die Verbindungen des Hohlkörpers im Adaptereinsatz bilden, angeordnet sind.

Hierdurch kann der Adaptereinsatz leichter mit PMMA-Knochenzement gefüllt werden, wodurch die Anwendbarkeit der Spacerform vereinfacht wird. Bevorzugt sind die Teile des Adaptereinsatzes formschlüssig miteinander verbindbar oder miteinander verbunden.

Um einen besonders kostengünstigen Aufbau für die Einmalverwendung zu erhalten, kann vorgesehen sein, dass das Basiselement, der Halbkugeleinsatz, der Adaptereinsatz und der Stempel aus Kunststoff gefertigt sind.

Die Verwendung von Kunststoff wird erst durch den erfindungsgemäßen Aufbau ermöglicht. Hierdurch kann die Spacerform als kostengünstiges Wegwerfprodukt angeboten werden. Eine Wiederverwertbarkeit ist aufgrund der notwendigen Hygiene beim Herstellen des Spacers möglichst auszuschließen.

Es wird erfindungsgemäß bevorzugt, wenn vorgesehen ist, dass die Ausnehmung des Basiselements einen geringeren Umfang hat als der Umfang des Halbkugeleinsatzes mit dem daran anliegenden Adaptereinsatz.

Hierdurch wird erreicht, dass der Halbkugeleinsatz und der Adaptereinsatz zusammengepresst werden, wenn sie aneinander anliegend in die Ausnehmung des Basiselements eingesteckt werden. Dadurch kann sichergestellt werden, dass die Schneidkante, die die Femurkopfform mit dem Hohlkörper verbindet, überschüssigen PMMA-Knochenzement abtrennt. Zudem wird sichergestellt, dass die mechanische Belastung, dem der Halbkugeleinsatz und der Adaptereinsatz ausgesetzt sind, wenn ein Kern zur Stabilisierung des Hüftspacers in den PMMA-Knochenzement, der in der Femurkopfform und dem Hohlkörper enthalten ist, hineingedrückt wird, ohne Verformung und ohne eine Trennung der beiden Einsätze voneinander aufgenommen werden kann.

Zur Vermeidung von Graten oder Unebenheiten auf der Gleitfläche kann vorgesehen sein, dass der Halbkugeleinsatz einteilig ausgebildet ist.

Hierdurch können störende Grate auf der Gleitfläche des Femurkopfs des Spacers vermieden werden. Solche Grate müssen ansonsten aufwendig entfernt werden, da sie bei einer Ablösung von Bruchstücken davon zu Entzündungen beziehungsweise zu Schwierigkeiten beim Heilungsprozess führen können.

Ferner kann vorgesehen sein, dass die Breite des Halbkugeleinsatzes, der die Femurkopfform an ihrem Rand umgibt, zwischen 5 mm und 45 mm breit ist.

Hierdurch können verschiedene Halbkugeleinsätze zur Ausformung unterschiedlich großer Femurköpfe eingesetzt werden. Hierdurch wird die Spacerform an verschiedene anatomische Verhältnisse anpassbar.

Mit einer Weiterbildung der Erfindung wird vorgeschlagen, dass in dem Stempel Entlüftungsöffnungen, insbesondere Entlüftungsbohrungen, vorgesehen sind, bevorzugt in der Stempelhohlform durchgehende Entlüftungsöffnungen vorgesehen sind.

Mit den Entlüftungsöffnungen beziehungsweise den Entlüftungsbohrungen können möglicherweise noch in der Spacerform zurückbleibende Lufteinschlüsse austreten. Die dadurch entstehenden Spitzen am Spacer müssen gegebenenfalls nach dem Entnehmen des Hüftspacers aus der Spacerform entfernt werden.

Erfindungsgemäße Spacerformen können sich dadurch auszeichnen, dass die Spacerform keine Einfüllöffnung zum Einfüllen von PMMA-Knochenzement aufweist.

Hierdurch kann eine aufwendige Befüllung der Spacerform mit Knochenzement vermieden werden. Der Vorteil der erfindungsgemäßen Spacerform liegt genau darin, dass keine aufwendige Befüllung durch eine solche Einfüllöffnung notwendig ist. Durch die offen zugänglichen Formen des Basiselements, des Halbkugeleinsatzes und des Adaptereinsatzes kann die erfindungsgemäße Spacerform leicht und auch von Laien befüllt werden.

Um eine bessere Variabilität der Spacerform zu erreichen, kann vorgesehen sein, dass die Spacerform wenigstens eine Einlage für die Hohlform und/oder die Stempelhohlform aufweist, die in die Hohlform und/oder die Stempelhohlform eingelegt ist oder einzulegen ist, so dass die Größe und/oder Form des Femurschafts des zu erzeugenden Hüftspacers mit der Einlage variierbar ist.

Hierdurch können mit der Spacerform verschiedene Femurschafte erzeugt werden. Ebenso kann es vorgesehen sein, dass verschiedene Halbkugeleinsätze und Adaptereinätze mit unterschiedlich geformten Femurkopfformen und Hohlkörpern vorhanden sind, mit denen unterschiedliche Hälse und Femurköpfe des Hüftspacers herstellbar sind. Bevorzugt sind die Adaptereinsätze mit dazu passenden Halbkugeleinsätzen kombinierbar. Insbesondere unterscheiden sich die Halbkugeleinsätze durch Femurkopfformen mit unterschiedlichem Durchmesser.

Die der Erfindung zugrundeliegenden Aufgaben werden auch gelöst durch ein Verfahren zur Herstellung eines Hüftspacers, mit den Schritten:
A) eine Femurkopfform eines Halbkugeleinsatzes und ein zweiseitig offener Hohlkörper eines Adaptereinsatzes werden mit PMMA-Knochenzement gefüllt und der Halbkugeleinsatz wird mit dem Adaptereinsatz zusammengesetzt;
B) danach werden der zusammengesetzte und mit PMMA-Knochenzement gefüllte Halbkugeleinsatz und Adaptereinsatz in eine Ausnehmung eines Basiselements eingesteckt, wobei dabei überschüssiger PMMA-Knochenzement mit Schneidkanten an der Verbindung zwischen der Femurkopfform und dem Hohlkörper abgetrennt wird, wobei das Basiselement eine Hohlform als Negativform eines Teils des zu erzeugenden Femurschafts des Hüftspacers aufweist, die nach dem Einstecken an die verbleibende freie Öffnung des Hohlkörpers des Adaptereinsatzes angrenzt;
C) anschließend wird ein Kopf eines Kerns zur mechanischen Stabilisierung des Hüftspacers in den PMMA-Knochenzement, der sich in dem Hohlkörper des mit dem Halbkugeleinsatz verbundenen Adaptereinsatzes befindet, eingesteckt, so dass ein Steg des Kerns in der Hohlform des Basiselements angeordnet ist;
D) die Hohlform des Basiselements wird im Überschuss mit PMMA-Knochenzement gefüllt;
E) anschließend wird ein Stempel mit einer Stempelhohlform als Negativform des restlichen Teils des zu erzeugenden Femurschafts des Hüftspacers auf den PMMA-Knochenzement in der Hohlform gedrückt, so dass die Femurkopfform, der Hohlkörper des Adaptereinsatzes, die Hohlform des Basiselements und die Stempelhohlform des Stempels die Form des zu erzeugenden Hüftspacers vorgeben, wobei beim Aufdrücken des Stempels überschüssiger PMMA-Knochenzement mit Schneidkanten an der Verbindung zwischen der Hohlform des Basiselements und der Stempelhohlform abgetrennt wird; und
F) anschließend, nach Aushärten des PMMA-Knochenzements in der Spacerform, die durch das Basiselement, der Halbkugeleinsatz, den Adaptereinsatz und den Stempel gebildet wird, wird der fertige Hüftspacer aus der Spacerform entnommen.

Es kann sowohl zuerst die Femurkopfform des Halbkugeleinsatzes und gegebenenfalls auch der Hohlkörper des Adaptereinsatzes mit PMMA-Knochenzement gefüllt werden und dann werden der Halbkugeleinsatz und der Adaptereinsatz zusammengesetzt, als auch zuerst der Halbkugeleinsatz und der Adaptereinsatz zusammengesetzt werden und dann anschließend die Femurkopfform des Halbkugeleinsatzes und der Hohlkörper des Adaptereinsatzes mit PMMA-Knochenzement gefüllt werden. Ferner kann auch erst die Femurkopfform mit PMMA-Knochenzement gefüllt werden, dann wird der Adaptereinsatz angefügt und anschließend die noch freien Volumina mit PMMA-Knochenzementteig durch die freie Öffnung des Hohlkörpers aufgefüllt (bevorzugt im Überschuss).

Der Steg ist mit dem Kopf des Kerns verbunden und bevorzugt einteilig ausgeführt.

Bevorzugt werden die Femurkopfform des Halbkugeleinsatzes und der Hohlkörper des Adaptereinsatzes im Überschuss mit PMMA-Knochenzement gefüllt. Dadurch kann sichergestellt werden, dass keine Hohlräume in dem Hohlkörper und in der Femurkopfform verbleiben, in denen kein PMMA-Knochenzement enthalten ist. Dadurch werden Hohlräume und Ausnehmungen in der Oberfläche des Hüftspacers vermieden.

Die Spacerform ist durch das Basiselement, den Halbkugeleinsatz, den Adaptereinsatz und den Stempel gebildet, beziehungsweise genauer durch die Hohlform des Basiselements, die Femurkopfform des Halbkugeleinsatzes, den Hohlkörper des Adaptereinsatzes und die Stempelhohlform des Stempels gebildet.

Bei erfindungsgemäßen Verfahren kann auch vorgesehen sein, dass als Kern zur mechanischen Stabilisierung des Hüftspacers ein Metallkern, insbesondere ein Stahlkern verwendet wird.

Diese Materialien weisen eine hohe Stabilität auf. Kerne (auch Armierungen genannt) zur Stabilisierung von Hüftspacern sind bekannt.

Besonders bevorzugt kann bei erfindungsgemäßen Verfahren vorgesehen sein, dass das Verfahren unter Verwendung einer erfindungsgemäßen Spacerform durchgeführt wird.

Des Weiteren kann vorgesehen sein, dass an dem Kern Abstandhalter angeordnet sind, wobei zumindest einer der Abstandhalter an der Hohlform des Basiselements anliegt, wenn der Kopf des Kerns in den Hohlkörper des mit dem Halbkugeleinsatz verbundenen Adaptereinsatzes eingesteckt wurde.

Bevorzugt bestehen die Abstandhalter aus PMMA. Ebenfalls bevorzugt erstrecken sich die Abstandhalter sternförmig radial vom Kern weg. Hierdurch wird erreicht, dass der Kern in der richtigen Position und Orientierung in der Spacerform angeordnet wird.

Schließlich wird für erfindungsgemäße Verfahren auch vorgeschlagen, dass nach dem Einstecken des Adaptereinsatzes mit dem Halbkugeleinsatz in die Ausnehmung des Basiselements der Adaptereinsatz von dem umlaufende Basiselement gegen den Halbkugeleinsatz gepresst wird, wobei bevorzugt der Adaptereinsatz gegen die Schneidkante des Halbkugeleinsatzes gepresst wird.

Der Halbkugeleinsatz und der Adaptereinsatz werden so miteinander fixiert, so dass sie sich nicht bei einer mechanischen Belastung, beispielsweise beim Einsetzen des Kerns, voneinander lösen oder gegeneinander bewegen. Zudem wird mit Hilfe der Schneidkanten eine Entgratung des zu erzeugenden Hüftspacers erreicht.

Der Erfindung liegt die überraschende Erkenntnis zugrunde, dass es durch den einfachen Aufbau und die Verwendung eines Stempels gelingt, die Spacerform auf einfach Weise durch große Öffnungen (die offene Hohlform, die offene Femurkopfform und der geöffnete Hohlkörper) mit PMMA-Knochenzement zu befüllen, ohne dass der PMMA-Knochenzement mit hohem Druck in die Spacerform eingepresst werden müsste. Hierdurch können auch hochviskose PMMA-Knochenzemente für die Herstellung des Hüftspacers verwendet werden. Durch die Aufnahme des Halbkugeleinsatzes und des Adaptereinsatzes in eine Ausnehmung des Basiselements wird auf einfache Weise eine Stabilisierung dieser Teile zueinander erreicht. Durch die Verwendung von Schneidkanten kann eine weitgehend gratfreie Oberfläche des Hüftspacers erzeugt werden.

Die Grundidee der Erfindung besteht darin, die Vorteile von Gießvorrichtungen, wie die hohe Maßhaltigkeit bei abgerundeten Formen, mit den Vorteilen von Prägevorrichtungen, wie Abtrennung von Graten und einfache Formgebung bei flachen Formen, zu kombinieren. Dies wird durch die Verwendung eines Stempels als Prägevorrichtung erreicht.

Das Basiselement fixiert Halbkugeleinsatz und den Adaptereinsatz und drückt diese aneinander an. Das ist auch für das Einsetzen des Kerns beziehungsweise des Metallkerns wichtig, damit die Einsätze (Halbkugeleinsatz und Adaptereinsatz) beim Einsetzen des Metallkerns nicht auseinander getrieben oder verformt werden. Dabei sind durch den erfindungsgemäßen Aufbau keine Klemmen, Clips oder andere Befestigungsmittel als Verschlusselemente notwendig, welche im OP-Alltag nur stören.

Zur Herstellung des Hüftspacers wird beispielsweise zunächst die "Halbkugel" der Femurkopfform des Halbkugeleinsatzes mit PMMA-Knochenzement vollgeschmiert. der zweiteilige Adaptereinsatz (in Form der weiteren "Halbkugelteile") wird eingesetzt und weiter mit PMMA-Knochenzement vollgefüllt. Bevorzugt wird erst der Adaptereinsatz gefüllt und dann mit Halbkugeleinsatz verbunden. Der Stempelschritt erfolgt als letztes. Das Stempeln kann auch als Prägeschritt bezeichnet werden.

Die erfindungsgemäße Spacerform hat einen modularen Aufbau. Sie muss nicht unter Druck befüllt werden, wodurch keine Verformung der Spacerform auftritt und es besteht auch keine Notwendigkeit für einen druckstabilen Aufbau der Spacerform.

Unter einem hochviskosen PMMA-Knochenzement werden solche PMMA-Knochenzemente verstanden, die eine nur kurze Anquellphase und eine rasche Klebfreiheit (innerhalb von weniger als 1,5 Minuten) aufweisen. Innerhalb der Verarbeitungsbreite ist die Viskosität lange Zeit gleichbleibend und zieht gegen Ende dieser Phase an. In der Regel ist die Verarbeitungsbreite vergleichsweise groß. Die Aushärtung erfolgt 1,5 bis 2 Minuten nach dem Ende der Verarbeitungsphase. Vergleiche hierzu Klaus-Dieter Kühn, "Knochenzemente für die Endoprohtetik", Springer-Verlag 2001 (ISBN 3-540-41182-8), dort insbesondere die Seiten 18 und 19. Zemente beziehungsweise Zementteige gelten auch als hochviskos, wenn sie 60 bis 90 Sekunden klebfrei bleiben. Die Viskosität selbst ist für die Definition nicht geeignet, da sie sich beim Aushärten des Knochenzements fortwährend verändert.

Anwendbare hochviskose Knochenzemente sind beispielsweise Palacos® R+G, Palacos® R der Firma Heraeus Medical GmbH, SmartSet® GHV der Firma DePuy Orthopädie GmbH, "Bone Cement" der Firma Biomet Deutschland GmbH.

Eine erfindungsgemäße Spacerform zur Herstellung von Hüftspacern kann beispielsweise zusammengesetzt sein aus
a) einem Basiselement, das eine Hohlform in Form eines halben Femurschafts enthält, wobei am proximalen Ende der Hohlform eine Ausnehmung angeordnet ist, die mit der Hohlform verbunden ist, und wobei am Rand der Hohlform eine die gesamte Hohlform des Femurschafts umlaufende Schneidkante angeordnet ist und wobei neben der Schneidkante das Basiselement in Bezug auf eine Ebene parallel zur Längsachse der Hohlform nach außen abfällt,
b) einen Halbkugeleinsatz, der in der Ausnehmung angeordnet ist beziehungsweise wird, der als eine Halbkugelform (als Femurkopfform) mit einem umlaufenden Rand ausgebildet ist, wobei die Halbkugeloberfläche der Halbkugelform entgegengesetzt zur Hohlform angeordnet ist beziehungsweise wird, wobei am Rand der Halbkugelform eine umlaufende Schneidkante angeordnet ist, und die Oberflächenkontur des umlaufenden Rands ausgehend von der Schneidkante nach außen abfällt,
c) einen Adaptereinsatz, der als Hohlkörper ausgebildet ist und in der Ausnehmung zwischen dem Halbkugeleinsatz und der Hohlform angeordnet ist beziehungsweise wird, wobei der Adaptereinsatz die Halbkugelform mit der Hohlform verbindet und
d) einen Stempel, der eine Stempelhohlform in Form eines halben Femurschafts enthält, der auf der Oberseite des Basiselements angeordnet ist beziehungsweise wird.

In die erfindungsgemäße Spacerform wird zur Armierung ein Stahlkern eingesetzt. Dieser Stahlkern wird durch sternförmige Abstandhalter von der Innenkontur der Spacerform, insbesondere der Hohlform des Basiselements und der Stempelhohlform des Stempels beabstandet, damit der PMMA-Knochenzementteig den Stahlkern vollständig umhüllen kann. Die Abstandhalter sind bevorzugt aus PMMA oder auch aus PMMA-Knochenzement gebildet.

Die erfindungsgemäße Vorrichtung enthält keine separaten Zuhaltevorrichtungen oder Verschlussvorrichtungen, wie Clips oder Schrauben.

Die Vorrichtung besitzt keine spezielle Einfüllöffnung für PMMA-Knochenzementteig.

Erfindungsgemäß kann vorgesehen sein, dass das Basiselement, der Halbkugeleinsatz, der Adaptereinsatz und der Stempel aus Kunststoff gefertigt sind.

Die Ausnehmung des Basiselements hat bevorzugt einen geringeren Umfang als der Umfang des Halbkugeleinsatzes mit dem daran anliegenden Adaptereinsatz. Dadurch wird nach Einsetzen des Halbkugeleinsatzes und des Adaptereinsatzes in die Ausnehmung des Basiselements eine Spannung erzeugt, mit der der Halbkugeleinsatz und der Adaptereinsatz gegeneinander gepresst werden. Dadurch trennt die Schneidkante am Halbkugeleinsatz den überschüssigen PMMA-Knochenzementteig vom PMMA-Knochenzementteig in dem aus den Halbkugeleinsatz und dem Adaptereinsatz gebildeten Formnest ab und drückt den überschüssigen PMMA-Knochenzement in den Hohlraum, der von der nach außen abfallenden Oberflächenkontur des Halbkugeleinsatzes und des Adaptereinsatzes gebildet wird. Eine Gratbildung am Kopf des Hüftspacers wird dadurch verhindert.

Der Halbkugeleinsatz ist bevorzugt einteilig ausgebildet. Dadurch gibt es keine Trennstelle auf der nach der Implantation tribologisch belasteten Gleitfläche der Kugel beziehungsweise des Kopfs des Hüftspacers. Trennstellen auf tribologisch belasteten Gleitflächen von Spacern können zu einem verstärkten Abrieb führen. Abriebpartikel von Spacern können Entzündungsprozesse verursachen.

Vorteilhat ist es, wenn der Rand des Halbkugeleinsatzes zwischen 5 mm und 45 mm breit ist. Dadurch ist es möglich, Halbkugelformen mit unterschiedlichen Durchmessern der Halbkugel herzustellen. Es wird bei größeren Durchmessern der Halbkugel nur der Rand beziehungsweise dessen Breite verringert und die äußeren Maße des Halbkugeleinsatzes bleiben konstant. Dadurch ist es möglich, Halbkugeleinsätze mit unterschiedlichen Halbkugeldurchmessern in die Ausnehmung des Basiselements einzusetzen, ohne dass die Form und Größe der Ausnehmung des Basiselements verändert werden muss. Der Durchmesser des Formnests des Halbkugelelements kann von 46 mm bis 75 mm variieren. Dadurch können mit der erfindungsgemäßen Spacerform Hüftspacer mit einem Kugeldurchmesser von 46 mm bis zu 75 mm hergestellt werden.

Weiterhin kann der Off-set des Hüftspacers grundsätzlich durch eine Kombination von Adaptereinsätzen mit unterschiedlicher Länge des und unter Verwendung von Halbkugeleinsätzen mit unterschiedlicher Breite des umlaufenden Randes variiert werden.

Erfindungsgemäß kann ferner vorgesehen sein, dass der Adaptereinsatz zweiteilig ausgebildet ist, wobei die beiden Formteile des Adaptereinsatzes formschlüssig miteinander verbunden sind.

Vorteilhaft ist es weiterhin, wenn der Stempel Entlüftungsbohrungen an seiner Oberseite besitzt.

Die erfindungsgemäße Spacerform hat den Vorteil, dass diese ohne Druckanwendung befüllt werden kann. Es sind große Zugänge zu den Formnestern vorhanden, so dass die Spacerform sowohl per Hand mittels Spatel als auch mit Zementierkartuschen befüllt werden kann. Besonders vorteilhaft ist dabei, dass bei Raumtemperatur hochviskoser PMMA-Zementteig benutzt werden kann, der bei Knie- und Hüft-TEP-Implantationen am häufigsten verwendet wird. Eine Vorkühlung von hochviskosem PMMA-Knochenzement, wie sie bei den bisherigen Gießformen notwendig ist, um den PMMA-Knochenzement ausreichend lange viskos genug zu halten, entfällt dadurch. Im Gegensatz zu den bekannten Gießvorrichtungen hat die erfindungsgemäße Vorrichtung keine Einfüllöffnung, durch die der PMMA-Knochenzementteig gepresst werden muss, wobei der Zementteig durch die ganze Gießvorrichtung gedrückt werden muss und dabei die enthaltene Luft verdrängt. Vorteilhaft ist weiterhin, dass durch die Schneidkanten überschüssiger PMMA-Zementteig vom Zementteig in den Formnestern abgeschnitten wird. Dadurch wird die Grat-Bildung minimiert beziehungsweise verhindert. Ein weiterer Vorteil der Vorrichtung ist, dass die Handhabung der Vorrichtung sehr einfach ist.

Erfindungsgemäß ist auch beispielsweise ein Verfahren zur Herstellung von Hüftspacern, bei dem
a) der Halbkugeleinsatz vollständig mit PMMA-Knochenzementteig ausgefüllt wird,
b) der Adaptereinsatz vollständig mit PMMA-Knochenzementteig ausgefüllt wird,
c) danach der mit Zementteig gefüllte Halbkugeleinsatz und der mit PMMA-Knochenzementteig ausgefüllte Adaptereinsatz zusammengepresst und in die Ausnehmung eingesteckt werden, wobei die Schneidkante des Halbkugeleinsatzes gegen die gegen die Wand des Adaptereinsatzes gepresst wird und überschüssiger PMMA-Knochenzementteig vom mit PMMA-Knochenzementteig gefüllten Innenraum des Halbkugeleinsatzes und des Adaptereinsatzes abgetrennt wird, und in den Hohlraum zwischen dem Halbkugeleinsatz und dem Adaptereinsatz gedrückt wird,
d) der Kopf des Stahlkerns in den PMMA-Knochenzementteig eingesteckt wird, der in dem Halbkugeleinsatz und dem Adaptereisatz angeordnet ist, wobei der Stahlkern mit den Abstandhaltern auf der Oberfläche der Hohlform aufliegt,
e) die Hohlform des Basiselements mit PMMA-Knochenzementteig gefüllt wird, bis diese vollständig gefüllt ist und der PMMA-Knochenzementteig über den Rand des Basiselements nach oben herausragt,
f) der Stempel auf die mit PMMA-Knochenzementteig gefüllte Hohlform des Basiselements gedrückt wird, wobei der Stempel gegen die Schneidkante des Basiselements gepresst wird und der überschüssige PMMA-Knochenzementteig vom Zementteig in der Hohlform abgetrennt und in den Raum zwischen dem nach außen abfallenden Basiselement und dem Stempel gedrückt wird, und dass
g) nach Aushärtung des PMMA-Knochenzementteigs der Hüftspacer aus der Spacerform entnommen wird.

Das erfindungsgemäße Verfahren stellt eine Kombination aus einem Gießverfahren und einem Prägeverfahren beziehungsweise Stempelverfahren dar. Vorteilhaft ist bei dem Verfahren, dass der PMMA-Knochenzement sowohl per Hand mit einem Spatel als auch mit einem Zementiersystem in die Spacerform ohne die Verwendung von Druck gefüllt werden kann. Dadurch ist es möglich, auch hochviskose PMMA-Knochenzemente, ohne vorherige Kühlung, bei Raumtemperatur zu verwenden. Vorteilhaft ist weiterhin, dass durch die drucklose Befüllung der Vorrichtung kein Auseinandertreiben der Spacerform, wie bei den bisher bekannten Gießformen auftreten kann. Dadurch sind aufwendige separate Zuhaltevorrichtungen, wie Clips und Schrauben, nicht notwendig. Der Verfahrensschritt (g) stellt eine Prägung der halben Schaftkontur dar. Ein Vorteil des Prägeschritts ist, dass Lufteinschlüsse zur Seite an dem Stempel vorbei aus der Spacerform heraus gedrückt werden.

Im Folgenden werden Ausführungsbeispiele der Erfindung anhand von zehn schematisch dargestellten Figuren erläutert, ohne jedoch dabei die Erfindung zu beschränken. Dabei zeigt:
Figur 1: eine schematische perspektivische Ansicht auf die Oberseite einer erfindungsgemäßen Spacerform;
Figur 2: eine schematische perspektivische Ansicht auf die Unterseite der Spacerform nach Figur 1;
Figur 3: eine schematische perspektivische Ansicht auf ein Basiselement der mit den Figuren 1 und 2 gezeigten Spacerform;
Figur 4: zwei schematische perspektivische Ansichten eines Stahlkerns zur Herstellung eines Hüftspacers;
Figur 5: eine schematische perspektivische Ansicht (links), eine Aufsicht (Mitte), eine geschnittene Ansicht (rechts oben) und eine weitere geschnittene Ansicht (rechts unten) eines Halbkugeleinsatzes der mit den Figuren 1 und 2 gezeigten Spacerform;
Figur 6: eine schematische perspektivische Ansicht auf das Basiselement nach Figur 3, bei dem der Halbkugeleinsatz nach Figur 5 und eine untere Hälfte eines Adaptereinsatzes nach Figur 8 eingesetzt ist sowie ein Stahlkern nach Figur 4 eingelegt ist;
Figur 7: eine schematische perspektivische Ansicht auf einen Stempel der mit den Figuren 1 und 2 gezeigten Spacerform;
Figur 8: eine schematische perspektivische Ansicht auf die zwei Teile eines Adaptereinsatzes der mit den Figuren 1 und 2 gezeigten Spacerform;
Figur 9: eine schematische perspektivische Ansicht eines Hüftspacers hergestellt mit einem erfindungsgemäßen Verfahren mit einer Spacerform nach den Figuren 1 und 2; und
Figur 10: eine schematische Seitenansicht einer erfindungsgemäßen Spacerform.

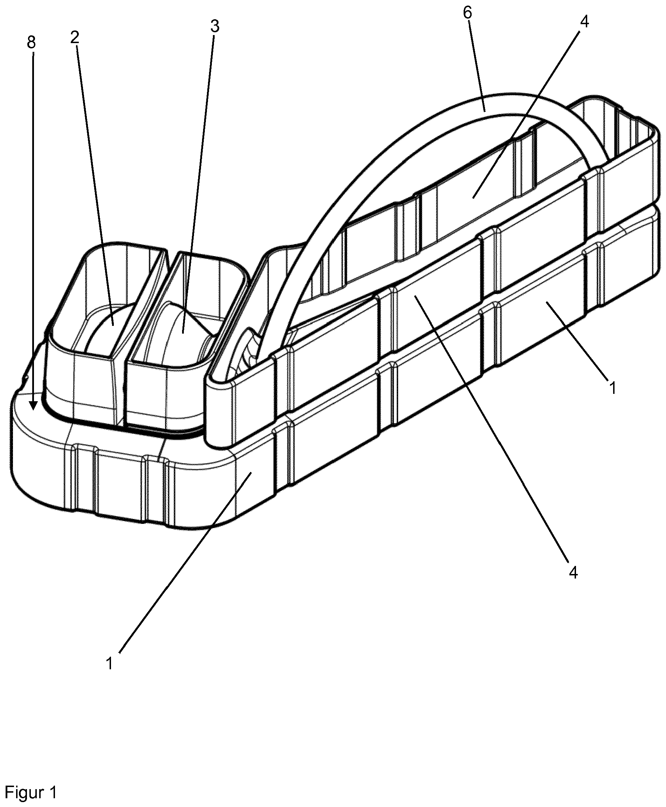

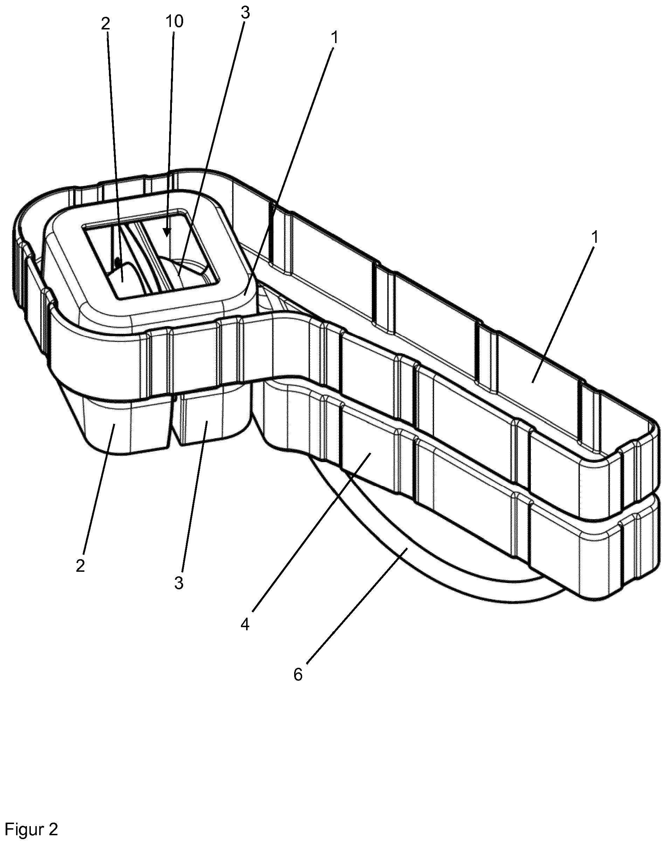

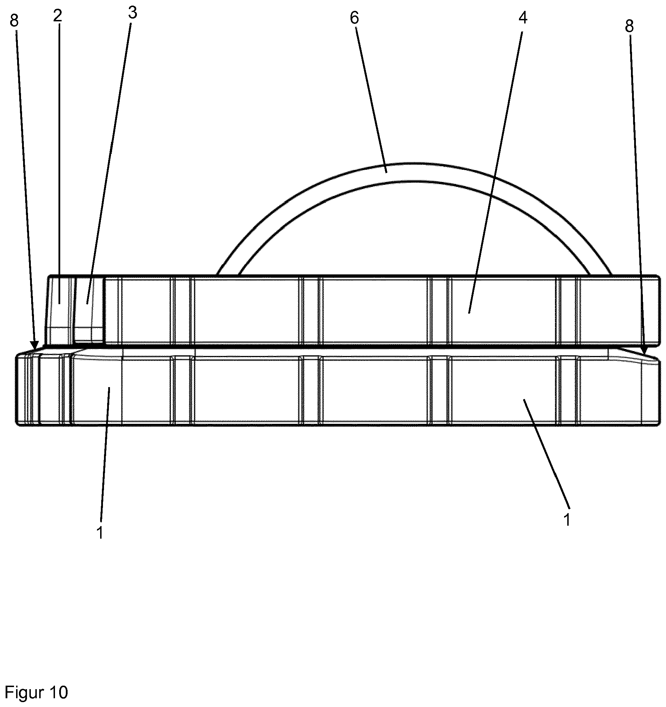

In den Figuren 1, 2 und 10 sind verschiedene schematische perspektivische Ansichten erfindungsgemäßer Spacerformen gezeigt. Figur 1 zeigt dabei eine perspektivische Sicht auf die Oberseite, Figur 2 zeigt eine perspektivische Sicht auf die Unterseite und Figur 10 zeigt eine Seitenansicht. Die Spacerform wird aus vier Teilen 1, 2, 3, 4 zusammengesetzt, nämlich einem Basiselement 1, einem Kugelkopfeinsatz 2, einem Adaptereinsatz 3 und einem Stempel 4. Das Basiselement 1, der Kugelkopfeinsatz 2, der Adaptereinsatz 3 und der Stempel 4 lassen sich lückenlos, das heißt passgenau zusammensetzen. Das Basiselement 1, der Kugelkopfeinsatz 2, der Adaptereinsatz 3 und der Stempel 4 bilden im zusammengesetzten Zustand in ihrem Inneren eine Form für den zu erzeugenden Spacer.

Das Basiselement 1, der Kugelkopfeinsatz 2, der Adaptereinsatz 3 und der Stempel 4 bestehen aus Kunststoff, können mit einem Spritzgussverfahren hergestellt werden und sind für den einmaligen Gebrauch bestimmt. An dem Stempel 4 ist ein Griff 6 befestigt, mit dem der Stempel 4 manuell auf das Basiselement 1 zu drücken ist, nachdem der Kugelkopfeinsatz 2 und der Adaptereinsatz 3 in eine Ausnehmung 10 in dem Basiselement 1 eingesetzt sind und nachdem der Kugelkopfeinsatz 2, der Adaptereinsatz 3, und eine Hohlform 12 (in den Figuren 1 und 2 nicht zu sehen) des Basiselements 1 mit PMMA-Knochenzement (nicht gezeigt) gefüllt wurden.

Der Stempel 4 wird dabei auf eine Schneidkante 14 (die in Figur 1 und 2 nicht zu sehen ist) am Rand der Hohlform 12 gedrückt, so dass hervorquellender PMMA-Knochenzement von dem PMMA-Knochenzement innerhalb der Spacerform abgeschnitten beziehungsweise abgekantet wird. Damit der abgeschnittene, hervor gepresste PMMA-Knochenzement von der Schneidkante 14 abfallen kann, weist das Basiselement 1 einen vom Rand der Hohlform 12 und von Rand der Ausnehmung 10 her abfallend geneigte umlaufende Fläche 8 auf. Da das Basiselement 1 mit der Unterseite (in Figur 2 gezeigt) auf einer ebenen Fläche, wie einem Tisch, aufgestellt werden kann, führt die Neigung der geneigten Fläche 8 dazu, dass der PMMA-Knochenzement von dieser Fläche 8 aufgrund der Gravitation ablaufen oder abrutschen kann.

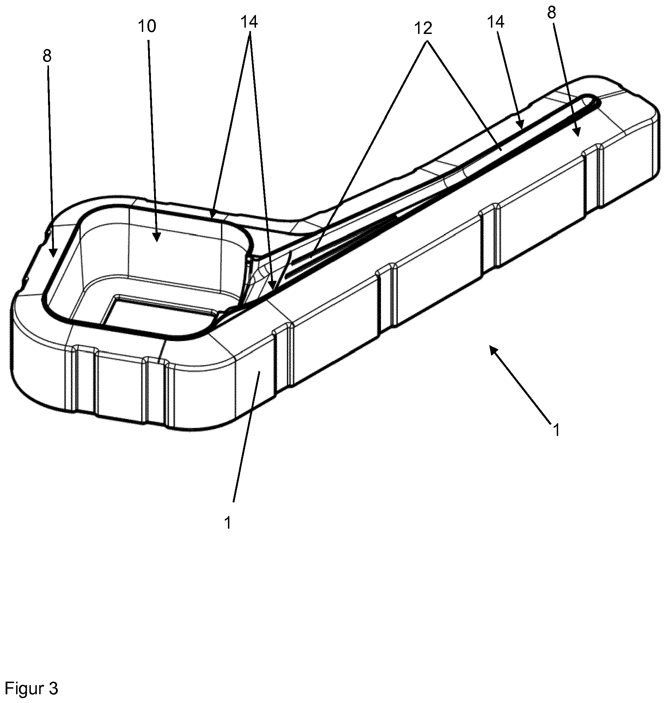

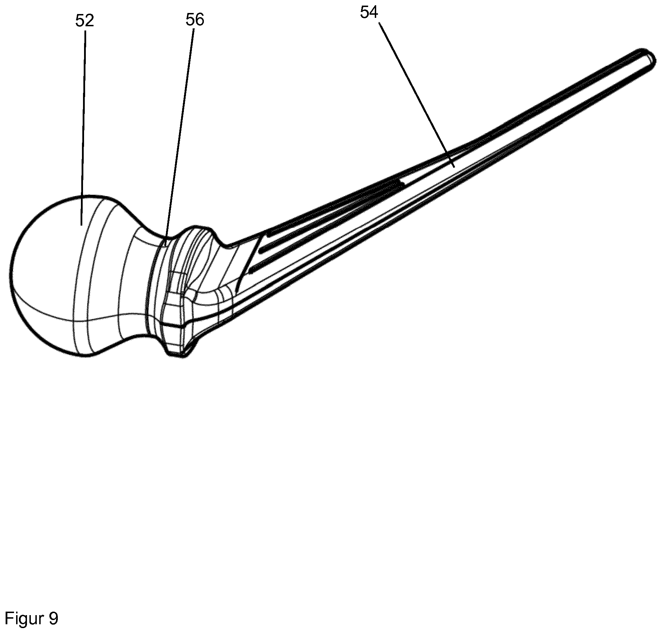

Figur 3 zeigt eine schematische perspektivische Ansicht auf das Basiselement 1 der Spacerform. In dem Basiselement 1 ist die Hohlform 12 zu erkennen, die ein Negativ des zu erzeugenden Femurschafts 54 des Hüftspacers ist (siehe Figur 9). Dazu wird die Hohlform 12 im Überschuss mit PMMA-Knochenzement gefüllt, nachdem der Kugelkopfeinsatz 2 und der Adaptereinsatz 3 in die Ausnehmung 10 eingesetzt wurden und bevor der Stempel 4 aufgedrückt wird. Der Rand der Hohlform 12 und der Ausnehmungen 10 auf der Oberseite des Basiselements 1 sind durch die umlaufende Schneidkanten 14 begrenzt. Die Schneidkanten 14 an der Ausnehmung 10 sind nicht notwendig, da an dieser Stelle kein PMMA-Knochenzement abgetrennt werden muss. Bevor die Hohlform 12 mit PMMA-Knochenzement im Überschuss gefüllt wird, wird ein Stahlkern (oder ein Kern aus einem anderen Material) zur mechanischen Stabilisierung des zu erzeugenden Hüftspacers in die Hohlform 12 eingelegt.

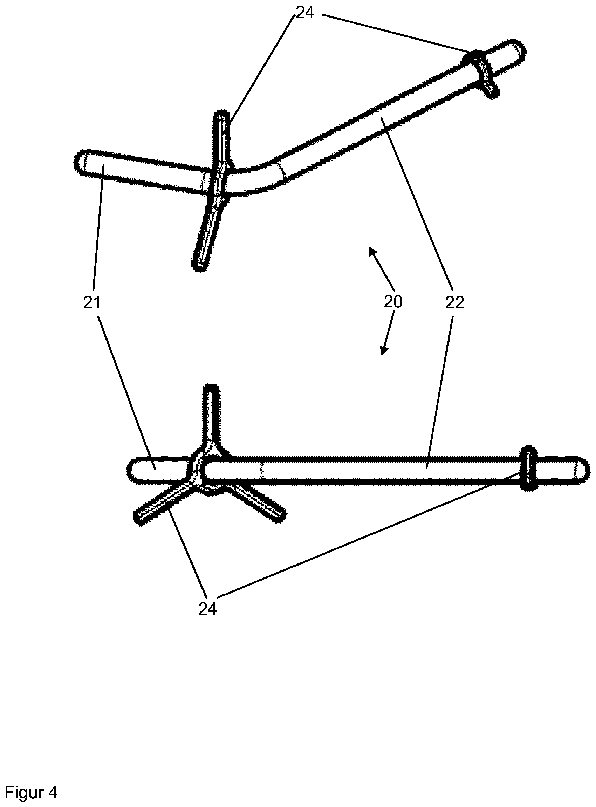

Figur 4 zeigt zwei schematische perspektivische Ansichten eines solchen Stahlkerns 20. Der Stahlkern 20 weist einen proximalen Kopf 21 und einen distalen Schaft 22 auf. Der Kopf 21 ist gegen den Schaft 22 abgewinkelt und mit diesem fest verbunden. Der Stahlkern 20 wird zur Herstellung beziehungsweise zur Armierung des herzustellenden Hüftspacers verwendet. Im Bereich des Knicks und im Bereich des distalen Endes des Schafts 22 des Kerns 20 sind zwei sternförmige Abstandhalter 24 aus PMMA vorgesehen, mit denen eine exakte Lagerung des Stahlkerns 20 in der Spacerform möglich ist. Dazu stützen sich die jeweils drei Arme der Abstandhalter 24 an den Innenwänden der Spacerform ab, die unter anderen durch die Hohlform 12 gebildet wird.

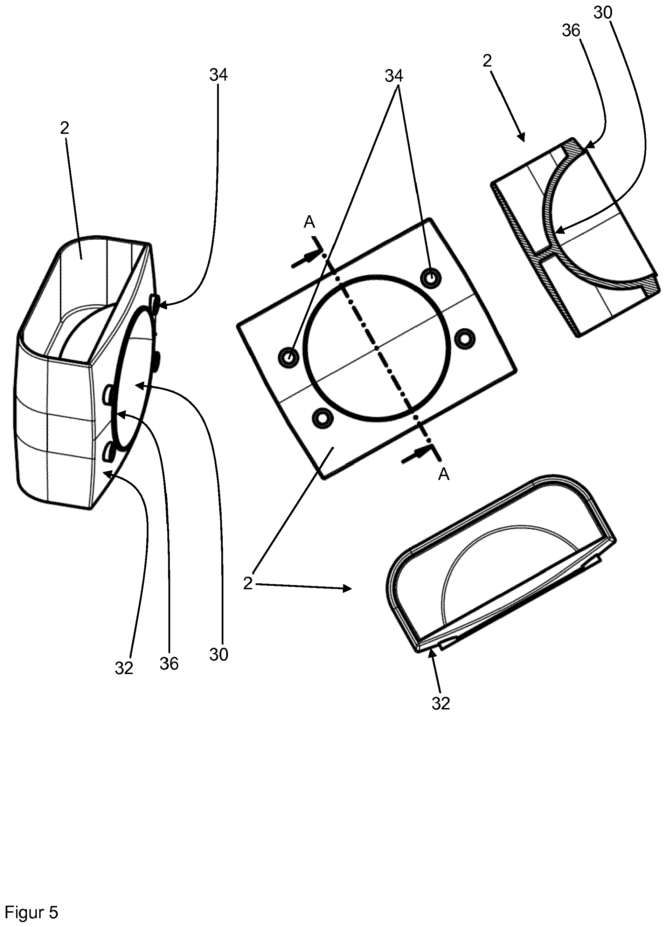

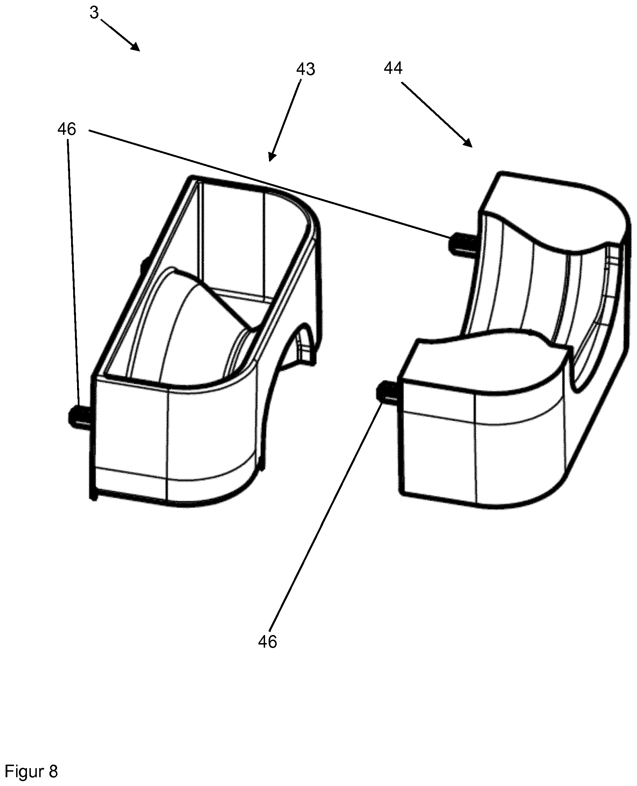

Figur 5 zeigt eine schematische perspektivische Ansicht (Figur 5 links), eine Aufsicht (Figur 5 Mitte), eine entlang der Ebene A-A bezüglich der Darstellung in der Mitte von Figur 5 geschnittene Ansicht (Figur 5 rechts oben) und eine weitere senkrecht zur Ebene A-A geschnittene Ansicht (Figur 5 rechts unten) des Halbkugeleinsatzes 2 der mit den Figuren 1, 2 und 10 gezeigten Spacerform. Der Halbkugeleinsatz 2 weist eine halbkugelförmige Femurkopfform 30 auf, die mit PMMA-Knochenzement gefüllt wird und die einen Teil der inneren Spacerform bildet. Mit dieser Femurkopfform 30 wird die Gleitfläche des Femurkopfs 52 des Hüftgelenkpacers erzeugt (siehe Figur 9). Daher ist die Femurkopfform 30 glatt und ohne Vorsprünge ausgebildet. Die zu der Femurkopfform 30 benachbarte Fläche 32 des Halbkugeleinsatzes 2 ist analog zum Basiselement 1 geneigt gestaltet, so dass austretender PMMA-Knochenzement abfallen kann beziehungsweise in den Zwischenraum zwischen dem Halbkugeleinsatz 2 und dem Adaptereinsatz 3 lösen kann. In dieser geneigten Fläche 32 sind vier Vertiefungen 34 als Befestigungselemente 34 vorgesehen. In diese Vertiefungen 34 sollen Stifte 46 des Adaptereinsatzes 3 (siehe Figur 8) greifen, um den Halbkugeleinsatz 2 und den Adaptereinsatz 3 miteinander in der korrekten Lage zu verbinden. Es kann vorgesehen sein, dass die Vertiefungen 34 durch eine eindeutige Form und/oder Lage nur auf eine einzige und eindeutige Art und Weise mit den Stiften 46 des Adaptereinsatzes 3 zu verbinden sind, um eine Fehlbedienung auszuschließen.

Die Femurkopfform 30 ist durch eine Schneidkante 36 begrenzt, mit der überschüssiger PMMA-Knochenzement beim Zusammensetzen des mit PMMA-Knochenzement gefüllten Halbkugeleinsatzes 2 mit dem ebenfalls mit PMMA-Knochenzement gefüllten Adaptereinsatz 3 abgetrennt wird.

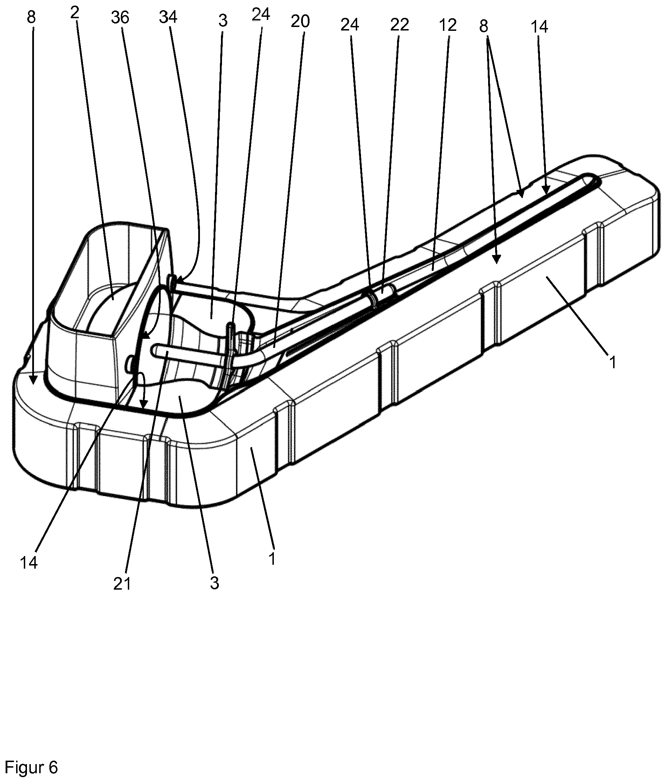

Figur 6 zeigt eine schematische perspektivische Ansicht auf das Basiselement 1 nach Figur 3, bei dem der Halbkugeleinsatz 2 nach Figur 5 und ein unterer Teil 44 eines Adaptereinsatzes 3 nach Figur 8 eingesetzt ist sowie ein Stahlkern 20 nach Figur 4 in die Hohlform 12 des Basiselements 1 eingelegt ist. In Figur 6 ist damit zu erkennen, wie der Stahlkern 20 in der Hohlform 12 und im Hohlkörper des Adaptereinsatzes 3 mit Hilfe der Abstandhalter 24 positioniert wird. Der in Figur 6 gezeigte Zustand wird bei der Herstellung des Hüftspacers so nicht realisiert, da der Kern 20 erst nach befüllen des Halbkugeleinsatzes 2 und des Adaptereinsatzes 3 mit PMMA-Knochenzement in den Adaptereinsatz 3 eingesteckt wird. Zudem kann auch vor dem Einlegen beziehungsweise Positionieren des Kerns 20 in der Hohlform 12 des Basiselements 1 bereits PMMA-Knochenzement in der Hohlform 12 eingefüllt worden sein, wobei nach dem Einsetzen des Kerns 20 der Kern 20 und damit die Hohlform 12 bevorzugt noch weiter mit PMMA-Knochenzement gefüllt wird, damit der Kern 20 vom PMMA-Knochenzement umhüllt wird.

Der untere Teil 44 des Adaptereinsatzes 3 ist mit dem Halbkugeleinsatz 2 verbunden, indem Stifte 46 (in Figur 6 nicht zu sehen) des Adaptereinsatzes 3 in die Vertiefungen 34 des Halbkugeleinsatzes 2 eingesteckt sind. Die Ausnehmung 10 hat einen etwas kleineren inneren Umfang als der Außenumfang des mit dem Adaptereinsatz 3 zusammengesetzten Halbkugeleinsatzes 2. Gleichzeitig ist der Kunststoff, aus dem das Basiselement 1 gefertigt ist, zumindest etwas elastisch, so dass der mit dem Adaptereinsatz 3 zusammengesetzte Halbkugeleinsatz 2 in die Ausnehmung 10 eingesteckt werden kann. Die Ausnehmung 10 hält dann den Halbkugeleinsatz 2 mit dem Adaptereinsatz 3 in Presspassung zusammen. Dabei wird der Adaptereinsatz 3 auf die Schneidkante 36 des Halbkugeleinsatzes 2 gepresst. Überstehender PMMA-Knochenzement kann so weitgehend frei von Graten abgetrennt werden.

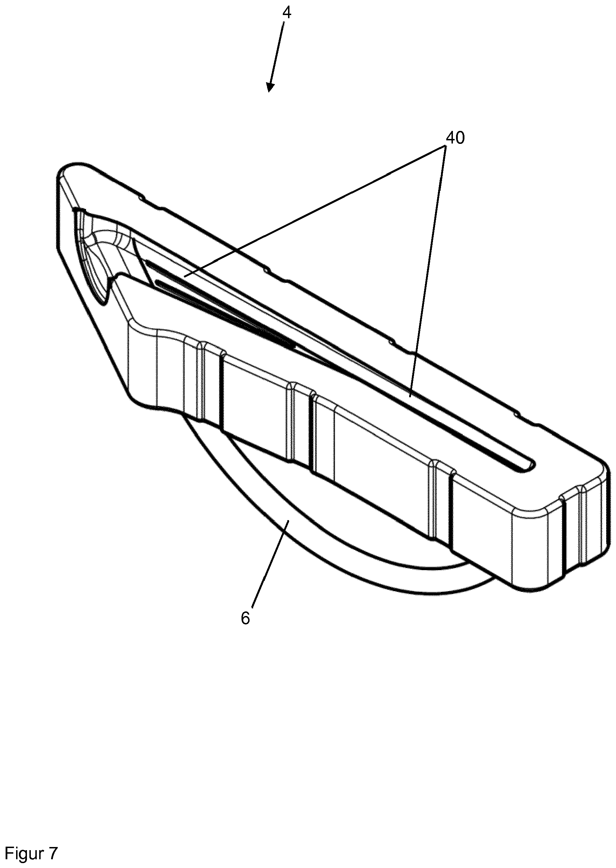

Figur 7 zeigt eine schematische perspektivische Ansicht auf einen Stempel 4 der mit den Figuren 1 und 2 gezeigten Spacerform. In dem Stempel 4 ist eine Stempelhohlform 40 vorgesehen, die ein Negativ des restlichen Femurschafts 54 des zu erzeugenden Hüftspacers bildet, der noch nicht durch die Hohlform 12 oder den Hohlkörper des Adaptereinsatzes 3 vorgegeben ist. Durch Aufdrücken des Stempels 4 wird der Rand der Stempelhohlform 40 auf die Schneidkante 14 des Basiselements 1 gepresst. Dabei wird aus der Spacerform austretender PMMA-Knochenzement abgetrennt beziehungsweise abgeschnitten, ohne dass Grate an der Verbindung verbleiben. Das Basiselement 1 und der Stempel 4 können Führungselemente (nicht gezeigt) aufweisen, die ein Aufdrücken des Stempels 4 auf das Basiselement 1 nur in einer Orientierung und/oder Lage ermöglichen.

In der Stempelhohlform 40 können mehrere durchgehende Bohrungen (nicht gezeigt) vorgesehen sein, durch die Luft beim Aufdrücken des Stempels 4 aus der Spacerform entweichen kann. An dieser Stelle entstehen an dem erzeugten Schaft des Hüftspacers dann Spitzen, die nach dem Aushärten und Entnehmen des Hüftspacers aus der Spacerform entfernt, also beispielsweise abgeschliffen werden müssen.

Es kann vorgesehen sein, dass verschiedene Einsätze (nicht gezeigt) für die Stempelhohlform 40 und die Hohlform 12 des Basiselements 1 vorhanden sind, die in die Stempelhohlform 40 beziehungsweise die Hohlform 12 des Basiselements 1 eingelegt werden können, um Hüftspacer mit unterschiedlich großen oder unterschiedlich geformten Femurschaften herstellen zu können. In der gleichen Weise können auch verschiedene Halbkugeleinsätze 2 und verschiedene Adaptereinsätze 3 vorgesehen sein und verwendet werden, um den Kopf des zu erzeugenden Hüftspacers variieren zu können (insbesondere dessen Durchmesser aber auch dessen Neigung gegen den Femurschaft). Zu allen diesen Varianten können auch unterschiedliche passende Stahlkerne 20 und passende Abstandhalter 24 vorgesehen sein. Dazu können die Abstandhalter 24 zunächst getrennt von den Stahlkernen 20 vorliegen und erst kurz vor dem Einsetzen die jeweils passenden Abstandhalter 24 aus PMMA auf die Stahlkerne 20 aufgeschoben werden.

Figur 8 zeigt eine schematische perspektivische Ansicht auf zwei Teile 43, 44 eines Adaptereinsatzes 3 der mit den Figuren 1 und 2 gezeigten Spacerform. Der untere Teil 44 des Adaptereinsatzes 3 ist bei der Anordnung nach Figur 6 in die Ausnehmung 10 des Basiselements 1 eingesetzt. Der obere Teil 43 und der untere Teil 44 des Adaptereinsatzes 3 können alternativ zu der gezeigten Ausführung über Schneidkanten (nicht gezeigt) an dem Rand des im Inneren des Adaptereinsatzes 3 gebildeten Hohlkörpers (wenn die beiden Teile 43, 44 zusammengesetzt sind) miteinander verbunden sein. Der Grat stört an dieser Stelle jedoch weniger als an anderen Stellen des zu erzeugenden Hüftspacers.

Sowohl am oberen Teil 43 als auch am unteren Teil 44 des Adaptereinsatzes 3 sind an der Öffnung beziehungsweise an der Verbindungsfläche zum Halbkugeleinsatz 2 jeweils zwei Stifte 46 als Befestigungselemente angeordnet, die in die Vertiefungen 34 des Halbkugeleinsatzes 2 eingesteckt werden können, um den Halbkugeleinsatz 2 mit dem Adaptereinsatz 3 zu verbinden.

Das Basiselement 1, der Kugelkopfeinsatz 2, der Adaptereinsatz 3 und der Stempel 4 bilden im zusammengesetzten Zustand in ihrem Inneren eine Form für den zu erzeugenden Spacer, also die erfindungsgemäße Spacerform.

Ein erfindungsgemäßes Verfahren kann mit der erfindungsgemäßen Spacerform beispielsweise wie folgt umgesetzt werden:
Es werden ein zur Behandlungssituation passender Halbkugeleinsatz 2 und ein Adaptereinsatz 3 ausgewählt. Die Femurkopfform 30 des Halbkugeleinsatzes 2 wird im Überschuss mit PMMA-Knochenzement gefüllt. Die beiden Teile 43, 44 des Adaptereinsatzes 3 werden über die Befestigungsmittel 34, 46 mit dem Halbkugeleinsatz 2 verbunden. Anschließend wird auch der Hohlkörper des Adaptereinsatzes 3 durch die verbleibende freie Öffnung mit PMMA-Knochenzement gefüllt. Alternativ kann der Hohlkörper des Adaptereinsatzes 3 auch vor dem zusammensetzen mit dem Halbkugeleinsatz 2 oder während dessen mit PMMA-Knochenzement gefüllt werden. Aufgrund der großen freien Querschnitte, lassen sich der Halbkugeleinsatz 2 und der Adaptereinsatz 3 bequem mit dem PMMA-Knochenzement füllen. Dies kann beispielsweise mit einem Spatel geschehen oder auch mit einem Knochenzementapplikator (nicht gezeigt). Es kann ein hochviskoser Knochenzement verwendet werden, um die Einsätze 2, 3 zu füllen.

Danach werden der aneinander befestigte Halbkugeleinsatz 2 und Adaptereinsatz 3, die mit PMMA-Knochenzement gefüllt sind, in die Ausnehmung 10 des Basiselements 1 eingesteckt. Da die Ausnehmung 10 im Innenumfang etwas kleiner ist als der Außenumfang der aneinander befestigten Einsätze 2, 3 werden der Halbkugeleinsatz 2 und der Adaptereinsatz 3 aufeinander gepresst und die Schneidkante 36 trennt austretenden überschüssigen PMMA-Knochenzement entlang der Verbindung der Femurkopfform 30 zum Hohlkörper des Adaptereinsatzes 3 ab. Aufgrund der geneigten Fläche 32 und des Zwischenraums kann sich der abgetrennte PMMA-Knochenzement von der Schnittstelle entfernen beziehungsweise abrutschen. Die Hohlform 12 des Basiselements 1, die als Negativform eines Teils des zu erzeugenden Femurschafts 54 des Hüftspacers (siehe Figur 9) vorgesehen ist, liegt im eingesetzten Zustand an die verbleibende freie Öffnung des Hohlkörpers des eingesteckten Adaptereinsatzes 3 an.

Zur Behandlungssituation passende Einsätze für die Hohlform 12 und für die Stempelhohlform 40 werden ausgewählt und in die Hohlform 12 beziehungsweise in die Stempelhohlform 40 eingelegt. Daraus ergibt sich dann eine durch die Einsätze modifizierte Hohlform 12 und eine modifizierte Stempelhohlform 40. Alternativ können auch die Hohlform 12 und die Stempelhohlform 40 in ihrer ursprünglichen Form verwendet werden. Ebenso können erfindungsgemäß auch verschiedene Basiselemente 1 und Stempel 4 mit unterschiedlichen Hohlformen 12 beziehungsweise Stempelhohlformen 40 vorgehalten werden, aus denen die zur Behandlungssituation passenden ausgewählt werden.

Anschließend wird ein Kopf 21 eines Kerns 20 zur mechanischen Stabilisierung des Hüftspacers in den PMMA-Knochenzement eingesteckt, der sich in dem Hohlkörper des Adaptereinsatzes 3 befindet, der (Adaptereinsatz 3) mit dem Halbkugeleinsatz 2 verbundenen ist, so dass ein Steg 22 des Kerns 20 in der Hohlform 12 des Basiselements 1 angeordnet ist. Zuvor kann bereits etwas PMMA-Knochenzement in die Hohlform 12 des Basiselements 1 eingefüllt worden sein. Die Hohlform 12 des Basiselements 1 wird im Überschuss mit PMMA-Knochenzement gefüllt, so dass der Kern 20 von PMMA-Knochenzement umhüllt ist. Abstandhalter 24 aus PMMA werden zur Positionierung des Kerns 20 in der Hohlform 12 und in dem Hohlkörper des Adaptereinsatzes 3 verwendet.

Anschließend wird der Stempel 4 mit der gegebenenfalls durch einen Einsatz modifizierten Stempelhohlform 40 als Negativform des restlichen Teils des zu erzeugenden Femurschafts 54 des Hüftspacers (siehe Figur 9) auf den fluiden PMMA-Knochenzement in der Hohlform 12 gedrückt. Dadurch geben die Femurkopfform 30, der Hohlkörper des Adaptereinsatzes 3, die Hohlform 12 des Basiselements 1 und die Stempelhohlform 40 des Stempels 4 die Form des zu erzeugenden Hüftspacers vor. Beim Aufdrücken des Stempels 4 wird überschüssiger PMMA-Knochenzement mit den Schneidkanten 14 an der Verbindung zwischen der Hohlform 12 des Basiselements 1 und der Stempelhohlform 40 abgetrennt. Aufgrund der geneigten Fläche 8 des Basiselements 1 kann sich der abgetrennte PMMA-Knochenzement von der Schnittstelle entfernen beziehungsweise abrutschen.

Nach Aushärten des PMMA-Knochenzements in der Spacerform, die durch das Basiselement 1, der Halbkugeleinsatz 2, den Adaptereinsatz 3 und den Stempel 4 gebildet wird, wird der fertige Hüftspacer aus der Spacerform entnommen. Noch vorhandene Grate und Spitzen (die durch Entlüftungsöffnungen in der Stempelhohlform 40 entstehen können), werden durch abschleifen entfernt. Der fertige Hüftspacer kann dann zur Behandlung eingesetzt werden.

Figur 9 zeigt eine schematische perspektivische Ansicht eines solchen erfindungsgemäßen Hüftspacers, der mit einem erfindungsgemäßen Verfahren mit einer Spacerform nach den Figuren 1 und 2 hergestellt wurde. Der Hüftspacer weist einen Kopf 52, einen Femurschaft 54 oder Schaft 54 und einen Hals 56 auf. Der Hals 56 verbindet den Schaft 54 mit dem Kopf 52 des Hüftspacers. Die proximale Oberfläche des Kopfs 52 bildet die Gleitfläche des Hüftgelenks und ist frei von Graten durch den Halbkugeleinsatz 2 geformt worden. Der Hals 56 und die angrenzenden Teile des Kopfs 52 und des Schafts 54 werden durch den Hohlkörper im Adaptereinsatz 3 geformt. Der restliche Schaft 54 wird von der Hohlform 12 im Basiselement 1 und von der Stempelhohlform 40 im Stempel 4 geformt.

Figur 10 zeigt eine schematische Seitenansicht einer erfindungsgemäßen Spacerform. In dieser Ansicht ist die Neigung der nach unten abgeneigten Fläche 8 neben dem Rand der Hohlform 12 und neben der Ausnehmung 10 gut zu erkennen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Basiselement
- 2: Halbkugeleinsatz
- 3: Adaptereinsatz
- 4: Stempel
- 6: Griff
- 8: Geneigte Fläche
- 10: Ausnehmung
- 12: Hohlform
- 14: Schneidkante
- 20: Stahlkern
- 21: Kopf
- 22: Steg
- 24: Abstandhalter
- 30: Femurkopfform
- 32: Geneigte Fläche
- 34: Vertiefung / Befestigungselement
- 36: Schneidkante
- 40: Stempelhohlform
- 43: Oberer Teil des Adaptereinsatzes
- 44: Unterer Teil des Adaptereinsatzes
- 46: Stift / Befestigungselement
- 52: Kopf des Hüftspacers
- 54: Femurschaft des Hüftspacers
- 56: Hals des Hüftspacers

## Patentansprüche

1. Spacerform zur Herstellung eines Hüftspacers, wobei die Spacerform die folgenden Teile aufweist:
ein Basiselement (1), in dem eine Hohlform (12) als Negativ einer Seite eines Femurschafts (54) des zu erzeugenden Spacers vorgesehen ist, wobei am proximalen Ende der Hohlform (12) im Basiselement (1) eine Ausnehmung (10) angeordnet ist, die mit der Hohlform (12) verbunden ist,
einen Halbkugeleinsatz (2), der eine Femurkopfform (30) als Negativ einer Seite eines Femurkopfs (52) des zu erzeugenden Spacers aufweist, wobei der Halbkugeleinsatz (2) in der Ausnehmung (10) im Basiselement (1) an einer der Verbindung zur Hohlform (12) gegenüberliegenden Seite der Ausnehmung (10) angeordnet ist oder anzuordnen ist, so dass die Femurkopfform (30) in Richtung zur Hohlform (12) ausgerichtet ist,
einen Adaptereinsatz (3), der als zweiseitig offener Hohlkörper ausgebildet ist und der in der Ausnehmung (10) des Basiselements (1) zwischen dem Halbkugeleinsatz (2) und der Hohlform (12) angeordnet ist oder anzuordnen ist, so dass die Ausnehmung (10) einen Druck auf den Adaptereinsatz (3) und den Halbkugeleinsatz (2) ausübt, wenn der Adaptereinsatz (3) und der Halbkugeleinsatz (2) in die Ausnehmung (10) eingesetzt sind, wobei der Hohlkörper des Adaptereinsatzes (3) die Femurkopfform (30) des Halbkugeleinsatzes (2) mit der Hohlform (12) im Basiselement (1) verbindet und
einen Stempel (4), der eine Stempelhohlform (40) in Form des Negativs des restlichen Femurschafts (54) aufweist und der auf der Oberseite des Basiselements (1) angeordnet ist oder aufzudrücken ist,
wobei am Rand der Hohlform (12) des Basiselements (1) und/oder am Rand der Stempelhohlform (40) des Stempels (4) eine die Verbindung der Hohlform (12) des Basiselements (1) zur Stempelhohlform (40) des Stempels (4) begrenzende Schneidkante (14) angeordnet ist und am Rand der Femurkopfform (30) und/oder an dem Rand der Öffnung des Hohlkörpers des Adaptereinsatzes (3) zur Femurkopfform (30) eine die Verbindung der Femurkopfform (30) zu dieser Öffnung des Hohlkörpers des Adaptereinsatzes (3) begrenzende Schneidkante (36) angeordnet ist.

2. Spacerform nach Anspruch 1, **dadurch gekennzeichnet, dass**
die Femurkopfform (30) einen umlaufenden Rand aufweist und die Oberflächenkontur (32) des umlaufenden Rands der Femurkopfform (30) des Halbkugeleinsatzes (2) ausgehend von der Femurkopfform (30) nach außen abfallend ist.

3. Spacerform nach Anspruch 1 oder 2, **dadurch gekennzeichnet, dass**
die Schneidkanten (14) die gesamte Hohlform (12) im Basiselement (1) begrenzen und/oder
die Schneidkanten (36) die gesamte Femurkopfform (30) im Halbkugeleinsatz (2) begrenzen und/oder der gesamte Hohlkörper des Adaptereinsatzes (3) in der Verbindung zur Femurkopfform (30) durch eine Schneidkante begrenzt ist.

4. Spacerform nach einem der vorangehenden Ansprüche, **dadurch gekennzeichnet, dass**
das Basiselement (1) neben der Hohlform (12) in Bezug auf einen Rand der Hohlform (12) nach außen abfällt.

5. Spacerform nach einem der vorangehenden Ansprüche, **dadurch gekennzeichnet, dass**
das Basiselement (1) an der Unterseite eine ebene Auflagefläche oder einen Standfuß zum Aufstellen der Spacerform auf einer ebenen Unterlage aufweist.

6. Spacerform nach einem der vorangehenden Ansprüche, **dadurch gekennzeichnet, dass**
der Adaptereinsatz (3) ein zweiteiliger oder mehrteiliger Adaptereinsatz (3) ist, wobei die Teile (43, 44) des Adaptereinsatzes (3) aneinander zu befestigen oder aneinander anlegbar sind, wobei bevorzugt an den Teilen (43, 44) des Adaptereinsatzes (3) Schneidkanten vorgesehen sind, wobei die Schneidkanten an den Rändern der den Hohlkörper bildenden Formen, die die Verbindungen des Hohlkörpers im Adaptereinsatz (3) bilden, angeordnet sind.

7. Spacerform nach einem der vorangehenden Ansprüche, **dadurch gekennzeichnet, dass**
das Basiselement (1), der Halbkugeleinsatz (2), der Adaptereinsatz (3) und der Stempel (4) aus Kunststoff gefertigt sind.

8. Spacerform nach einem der vorangehenden Ansprüche, **dadurch gekennzeichnet, dass**
die Ausnehmung (10) des Basiselements (1) einen geringeren Umfang hat als der Umfang des Halbkugeleinsatzes (2) mit dem daran anliegenden Adaptereinsatz (3).

9. Spacerform nach einem der vorangehenden Ansprüche, **dadurch gekennzeichnet, dass**
der Halbkugeleinsatz (2) einteilig ausgebildet ist.

10. Spacerform nach einem der vorangehenden Ansprüche, **dadurch gekennzeichnet, dass**
die Breite des Halbkugeleinsatzes (2), der die Femurkopfform (30) an ihrem Rand umgibt, zwischen 5 mm und 45 mm breit ist.

11. Spacerform nach einem der vorangehenden Ansprüche, **dadurch gekennzeichnet, dass**
in dem Stempel (4) Entlüftungsöffnungen, insbesondere Entlüftungsbohrungen, vorgesehen sind, bevorzugt in der Stempelhohlform (40) durchgehende Entlüftungsöffnungen vorgesehen sind.

12. Spacerform nach einem der vorangehenden Ansprüche, **dadurch gekennzeichnet, dass**
die Spacerform keine Einfüllöffnung zum Einfüllen von PMMA-Knochenzement aufweist.

13. Spacerform nach einem der vorangehenden Ansprüche, **dadurch gekennzeichnet, dass**
die Spacerform wenigstens eine Einlage für die Hohlform (12) und/oder die Stempelhohlform (40) aufweist, die in die Hohlform (12) und/oder die Stempelhohlform (40) eingelegt ist oder einzulegen ist, so dass die Größe und/oder Form des Femurschafts (54) des zu erzeugenden Hüftspacers mit der Einlage variierbar ist.

14. Verfahren zur Herstellung eines Hüftspacers, **gekennzeichnet durch** die Schritte eine Femurkopfform (30) eines Halbkugeleinsatzes (2) und ein zweiseitig offener Hohlkörper eines Adaptereinsatzes (3) werden mit PMMA-Knochenzement gefüllt und der Halbkugeleinsatz (2) wird mit dem Adaptereinsatz (3) zusammengesetzt;
danach werden der zusammengesetzte und mit PMMA-Knochenzement gefüllte Halbkugeleinsatz (2) und Adaptereinsatz (3) in eine Ausnehmung (10) eines Basiselements (1) eingesteckt, wobei dabei überschüssiger PMMA-Knochenzement mit Schneidkanten (36) an der Verbindung zwischen der Femurkopfform (30) und dem Hohlkörper abgetrennt wird, wobei das Basiselement (1) eine Hohlform (12) als Negativform eines Teils des zu erzeugenden Femurschafts (54) des Hüftspacers aufweist, die nach dem Einstecken an die verbleibende freie Öffnung des Hohlkörpers des Adaptereinsatzes (3) angrenzt;
anschließend wird ein Kopf (21) eines Kerns (20) zur mechanischen Stabilisierung des Hüftspacers in den PMMA-Knochenzement, der sich in dem Hohlkörper des mit dem Halbkugeleinsatz (2) verbundenen Adaptereinsatzes (3) befindet, eingesteckt, so dass ein Steg (22) des Kerns (20) in der Hohlform (12) des Basiselements (1) angeordnet ist;
die Hohlform (12) des Basiselements (1) wird im Überschuss mit PMMA-Knochenzement gefüllt;
anschließend wird ein Stempel (4) mit einer Stempelhohlform (40) als Negativform des restlichen Teils des zu erzeugenden Femurschafts (54) des Hüftspacers auf den PMMA-Knochenzement in der Hohlform (12) gedrückt, so dass die Femurkopfform (30), der Hohlkörper des Adaptereinsatzes (3), die Hohlform (12) des Basiselements (1) und die Stempelhohlform (40) des Stempels (4) die Form des zu erzeugenden Hüftspacers vorgeben, wobei beim Aufdrücken des Stempels (4) überschüssiger PMMA-Knochenzement mit Schneidkanten (14) an der Verbindung zwischen der Hohlform (12) des Basiselements (1) und der Stempelhohlform (40) abgetrennt wird; und
anschließend, nach Aushärten des PMMA-Knochenzements in der Spacerform, die **durch** das Basiselement (1), der Halbkugeleinsatz (2), den Adaptereinsatz (3) und den Stempel (4) gebildet wird, wird der fertige Hüftspacer aus der Spacerform entnommen.

15. Verfahren nach Anspruch 14, **dadurch gekennzeichnet, dass** als Kern (20) zur mechanischen Stabilisierung des Hüftspacers ein Metallkern (20), insbesondere ein Stahlkern (20) verwendet wird.

16. Verfahren nach einem der Ansprüche 14 oder 15, **dadurch gekennzeichnet, dass** das Verfahren unter Verwendung einer Spacerform nach einem der Ansprüche 1 bis 13 durchgeführt wird.

17. Verfahren nach einem der Ansprüche 14 bis 16, **dadurch gekennzeichnet, dass** an dem Kern (20) Abstandhalter (24) angeordnet sind, wobei zumindest einer der Abstandhalter (24) an der Hohlform (12) des Basiselements (1) anliegt, wenn der Kopf (21) des Kerns (20) in den Hohlkörper des mit dem Halbkugeleinsatz (2) verbundenen Adaptereinsatzes (3) eingesteckt wurde.

18. Verfahren nach einem der Ansprüche 14 bis 17, **dadurch gekennzeichnet, dass** nach dem Einstecken des Adaptereinsatzes (3) mit dem Halbkugeleinsatz (2) in die Ausnehmung (10) des Basiselements (1) der Adaptereinsatz (3) von dem umlaufende Basiselement (1) gegen den Halbkugeleinsatz (2) gepresst wird, wobei bevorzugt der Adaptereinsatz (3) gegen die Schneidkante (36) des Halbkugeleinsatzes (2) gepresst wird.

## Claims

1. Spacer mould for producing a hip spacer, whereby the spacer mould comprises the following parts:
a base element (1), in which a hollow mould (12) is provided as negative image of one side of a femoral stem (54) of the spacer to be generated, whereby a recess (10) is arranged on the proximal end of the hollow mould (12) in the base element (1) and is connected to the hollow mould (12);
a semi-spherical insert (2) comprising a femoral head mould (30) as negative image of one side of a femoral head (52) of the spacer to be generated, whereby the semi-spherical insert (2) is or can be arranged in the recess (10) in the base element (1) at a side of the recess (10) that is opposite to the connection to the hollow mould (12), such that the femoral head mould (30) is aligned in the direction towards the hollow mould (12);
an adapter insert (3) that is designed as a hollow body that is open on two sides and that is or can be arranged in the recess (10) of the base element (1) between the semi-spherical insert (2) and the hollow mould (12) such that the recess (10) exerts a pressure onto the adapter insert (3) and the semi-spherical insert (2),
when the adapter insert (3) and the semi-spherical insert (2) are inserted into the recess (10), whereby the hollow body of the adapter insert (3) connects the femoral head mould (30) of the semi-spherical insert (2) to the hollow mould (12) in the base element (1); and
a punch (4) that comprises a punch hollow mould (40) in the form of the negative image of the remaining femoral stem (54) and that is arranged on or can be pressed onto the top side of the base element (1);
whereby a cutting edge (14) bounding the connection of the hollow mould (12) of the base element (1) to the punch hollow mould (40) of the punch (4) is arranged on the edge of the hollow mould (12) of the base element (1) and/or on the edge of the punch hollow mould (40) of the punch (4), and a cutting edge (36) bounding the connection of the femoral head mould (30) to the opening of the hollow body of the adapter insert (3) is arranged on the edge of the femoral head mould (30) and/or on the edge of said opening of the hollow body of the adapter insert (3) to the femoral head mould (30).

2. Spacer mould according to claim 1, **characterised in that**
the femoral head mould (30) comprises a circumferential edge and the surface contour (32) of the circumferential edge of the femoral head mould (30) of the semi-spherical insert (2) slopes towards the outside originating from the femoral head mould (30).

3. Spacer mould according to claim 1 or 2, **characterised in that**
the cutting edges (14) bound the entire hollow mould (12) in the base element (1) and/or
the cutting edges (36) bound the entire femoral head mould (30) in the semi-spherical insert (2) and/or the entire hollow body of the adapter insert (3), in the connection to the femoral head mould (30), is bounded by a cutting edge.

4. Spacer mould according to any one of the preceding claims, **characterised in that** the base element (1), next to the hollow mould (12), slopes towards the outside with respect to an edge of the hollow mould (12).

5. Spacer mould according to any one of the preceding claims, **characterised in that** the base element (1) comprises, on the bottom side, a planar support surface or a pedestal for set-up of the spacer mould on a planar support.

6. Spacer mould according to any one of the preceding claims, **characterised in that** the adapter insert (3) is a two-part or multi-part adapter insert (3), whereby the parts (43, 44) of the adapter insert (3) can be attached to each other or placed against each other, whereby, preferably, cutting edges are provided on the parts (43, 44) of the adapter insert (3), whereby the cutting edges are arranged on the edges of the moulds forming the hollow body that form the connections of the hollow body in the adapter insert (3).

7. Spacer mould according to any one of the preceding claims, **characterised in that** the base element (1), the semi-spherical insert (2), the adapter insert (3), and the punch (4) are fabricated from plastic material.

8. Spacer mould according to any one of the preceding claims, **characterised in that** the recess (10) of the base element (1) has a smaller circumference than the circumference of the semi-spherical insert (2) with the adapter insert (3) touching against it.

9. Spacer mould according to any one of the preceding claims, **characterised in that** the semi-spherical insert (2) is provided as a single part.

10. Spacer mould according to any one of the preceding claims, **characterised in that** the width of the semi-spherical insert (2) surrounding the edge of the femoral head mould (30) is between 5 mm and 45 mm wide.

11. Spacer mould according to any one of the preceding claims, **characterised in that** ventilation openings, in particular ventilation bore holes, are provided in the punch (4), preferably **in that** through-going ventilation openings are provided in the punch hollow mould (40).

12. Spacer mould according to any one of the preceding claims, **characterised in that** the spacer mould comprises no filling opening for filling-in PMMA bone cement.

13. Spacer mould according to any one of the preceding claims, **characterised in that** the spacer mould comprises at least one insert for the hollow mould (12) and/or the punch hollow mould (40) that is or can be inserted into the hollow mould (12) and/or the punch hollow mould (40) such that the size and/or the shape of the femoral stem (54) of the hip spacer to be generated can be varied by means of the insert.

14. Method for producing a hip spacer, **characterised by** the steps of filling a femoral head mould (30) of a semi-spherical insert (2) and a hollow body, open on two sides, of an adapter insert (3) with PMMA bone cement and assembling the semi-spherical insert (2) and the adapter insert (3);
followed by inserting the assembled and PMMA bone cement-filled semi-spherical insert (2) and adapter insert (3) into a recess (10) of a base element (1), whereby an excess of PMMA bone cement is severed by cutting edges (36) at the connection between the femoral head mould (30) and the hollow body, whereby the base element (1) comprises a hollow mould (12) as negative mould of a part of the femoral stem (54) of the hip spacer to be generated, whereby the hollow mould, after being plugged in, is situated adjacent to the remaining free opening of the hollow body of the adapter insert (3);
followed by plugging a head (21) of a core (20) for mechanical stabilisation of the hip spacer into the PMMA bone cement present in the hollow body of the adapter insert (3) connected to the semi-spherical insert (2), such that a fin (22) of the core (20) is arranged in the hollow mould (12) of the base element (1); filling the hollow mould (12) of the base element (1) with an excess of PMMA bone cement;
followed by pushing a punch (4) with a punch hollow mould (40) as negative mould of the remaining part of the femoral stem (54) of the hip spacer to be generated onto the PMMA bone cement in the hollow mould (12), such that the femoral head mould (30), the hollow body of the adapter insert (3), the hollow mould (12) of the base element (1), and the punch hollow mould (40) of the punch (4) define the shape of the hip spacer to be generated, whereby an excess of PMMA bone cement is severed by cutting edges (14) at the connection between the hollow mould (12) of the base element (1) and the punch hollow mould (40) while the punch (4) is being pushed on; and
followed by taking the finished hip spacer out of the spacer mould after the PMMA bone cement has cured in the spacer mould formed by the base element (1), the semi-spherical insert (2), the adapter insert (3), and the punch (4).

15. Method according to claim 14, **characterised in that**
a metal core (20), in particular a steel core (20), is used as core (20) for mechanical stabilisation of the hip spacer.

16. Method according to any one of the claims 14 or 15, **characterised in that**
the method is performed using a spacer mould according to any one of the claims 1 to 13.

17. Method according to any one of the claims 14 to 16, **characterised in that** distance pieces (24) are arranged on the core (20), whereby at least one of the distance pieces (24) touches against the hollow mould (12) of the base element (1) after the head (21) of the core (20) has been plugged into the hollow body of the adapter insert (3) connected to the semi-spherical insert (2).

18. Method according to any one of the claims 14 to 17, **characterised in that**
the adapter insert (3) is pressed against the semi-spherical insert (2) by the circumferential base element (1) after the adapter insert (3) and the semi-spherical insert (2) are plugged into the recess (10) of the base element (1), whereby the adapter insert (3) preferably is pressed against the cutting edge (36) of the semi-spherical insert (2).

## Revendications

1. Moule de fabrication de spacers pour la fabrication d'un spacer de hanche, le moule de fabrication de spacers présentant les composants suivants :
un élément de base (1), dans lequel est prévu une forme creuse (12) en tant que négatif d'un côté d'une tige fémorale (54) du spacer à fabriquer, de sorte que soit agencé à l'extrémité proximale de la forme creuse (12) dans l'élément de base (1) un évidement (10), qui est relié à la forme creuse (12),
un insert en demi-sphère (2) qui présente une forme de tête fémorale (30) en tant que négatif d'une tête fémorale (52) du spacer à fabriquer, l'insert en demi-sphère (2) étant agencé ou devant être agencé dans l'évidement (10) dans l'élément de base (1) sur un côté de l'évidement (10) opposé à la liaison vers la forme creuse (12), de sorte que la forme de tête fémorale (30) soit orientée dans le sens vers la forme creuse (12),
un insert adaptateur (3), qui est formé en tant que corps creux ouvert bilatéralement et qui est agencé ou qui doit être agencé dans l'évidement (10) de l'élément de base (1) entre l'insert en demi-sphère (2) et la forme creuse (12), de sorte que l'évidement (10) exerce une pression sur l'insert adaptateur (3) et l'insert en demi-sphère (2), lorsque l'insert adaptateur (3) et
l'insert en demi-sphère (2) sont en place dans l'évidement (10), le corps creux de l'insert adaptateur (3) reliant la forme de tête fémorale (30) de l'insert en demi-sphère (2) avec la forme creuse (12) dans l'élément de base (1) et
une étampe (4), qui présente une forme creuse d'étampe (40) sous forme de négatif du reste de la tige fémorale (54) et qui est agencée ou qui doit être engagée par pression sur le côté supérieur de l'élément de base (1), un bord coupant (14) qui limite la liaison de la forme creuse (12) de l'élément de base (1) vers la forme creuse d'étampe (40) de l'étampe (4) étant agencé au bord de la forme creuse (12) de l'élément de base (1) et/ou au bord de la forme creuse d'étampe (40) de l'étampe (4) et un bord coupant (36) qui limite la liaison de la forme de tête fémorale (30) vers cette ouverture du corps creux de l'insert adaptateur (3) étant agencé au bord de la forme de tête fémorale (30) et/ou au bord de l'ouverture du corps creux de l'insert adaptateur (3) vers la forme de tête fémorale (30).

2. Moule de fabrication de spacers selon la revendication 1, **caractérisé en ce, que** la forme de tête fémorale (30) présente un bord périphérique et que le contour de surface (32) du bord périphérique de la forme de tête fémorale (30) de l'insert en demi-sphère (2) soit incliné vers le bas en partant de la forme de tête fémorale (30).

3. Moule de fabrication de spacers selon la revendication 1 ou 2, **caractérisé en ce, que** les bords coupants (14) limitent l'ensemble de la forme creuse (12) dans l'élément de base (1) et/ou
les bords coupants (36) limitent l'ensemble de la forme de tête fémorale (30) dans l'insert en demi-sphère (2) et/ou que l'ensemble du corps creux de l'insert adaptateur (3) dans la liaison de la forme de tête fémorale (30) soit limité par un bord coupant.

4. Moule de fabrication de spacers selon l'une des revendications précédentes, **caractérisé en ce, que**
l'élément de base (1) à côté de la forme creuse (12) soit incliné vers le bas par rapport à un bord de la forme creuse (12).

5. Moule de fabrication de spacers selon l'une des revendications précédentes, **caractérisé en ce, que**
l'élément de base (1) présente sur la face inférieure une surface d'appui plane ou un pied-support pour installer le moule de fabrication de spacers sur un support plan.

6. Moule de fabrication de spacers selon l'une des revendications précédentes, **caractérisé en ce, que**
l'insert adaptateur (3) soit un insert adaptateur (3) en deux ou en plusieurs parties, les composants (43, 44) de l'insert adaptateur (3) pouvant être fixés entre eux ou pouvant être appliqués l'un contre l'autre, des bords coupants étant de préférence prévus aux composants (43, 44) de l'insert adaptateur (3), de sorte que les bords coupants soient agencés aux bords des formes formant le corps creux qui forment les liaisons du corps creux dans l'insert adaptateur (3).

7. Moule de fabrication de spacers selon l'une des revendications précédentes, **caractérisé en ce, que**
l'élément de base (1), l'insert en demi-sphère (2), l'insert adaptateur (3) et l'étampe (4) soient fabriqués en matière plastique.

8. Moule de fabrication de spacers selon l'une des revendications précédentes, **caractérisé en ce, que**
l'évidement (10) de l'élément de base (1) a une circonférence plus petite que la circonférence de l'insert en demi-sphère (2) avec l'insert adaptateur (3) adjacent.

9. Moule de fabrication de spacers selon l'une des revendications précédentes, **caractérisé en ce, que**
l'insert en demi-sphère (2) soit formé en une seule pièce.

10. Moule de fabrication de spacers selon l'une des revendications précédentes, **caractérisé en ce, que**
la largeur de l'insert en demi-sphère (2), qui entoure avec son bord la forme de tête fémorale (30), mesure entre 5 mm et 45 mm.

11. Moule de fabrication de spacers selon l'une des revendications précédentes, **caractérisé en ce, que**
soient prévus dans l'étampe (4) des orifices d'évacuation d'air, notamment des trous d'évacuation d'air, de préférence des orifices traversants d'évacuation d'air dans la forme creuse d'étampe (40).

12. Moule de fabrication de spacers selon l'une des revendications précédentes, **caractérisé en ce, que**
le moule de fabrication de spacers ne comporte pas d'orifice de remplissage pour le remplissage de ciment osseux à base de PMMA.

13. Moule de fabrication de spacers selon l'une des revendications précédentes, **caractérisé en ce, que**
le moule de fabrication de spacers présente au moins une pièce intercalaire pour la forme creuse (12) et/ou pour la forme creuse d'étampe (40) qui soit positionnée ou qui doit être positionnée dans la forme creuse (12) et/ou dans la forme creuse d'étampe (40), de sorte que la taille et/ou la forme de la tige fémorale (54) du spacer de hanche à fabriquer soit variable.

14. Procédé de fabrication d'un spacer de hanche, **caractérisé par** les étapes suivantes :
une forme de tête fémorale (30) d'un insert en demi-sphère (2) et un corps creux ouvert bilatéralement d'un insert adaptateur (3) sont remplis de ciment osseux à base de PMMA et l'insert en demi-sphère (2) est assemblé avec l'insert adaptateur (3) ;
l'insert en demi-sphère (2) et l'insert adaptateur (3) assemblés et remplis de ciment osseux à base de PMMA sont ensuite insérés dans un évidement (10) d'un élément de base (1), le ciment osseux à base de PMMA excédentaire étant alors coupé par les bords coupants (36) au niveau de la liaison entre la forme de tête fémorale (30) et le corps creux, l'élément de base (1) présentant une forme creuse (12) en tant que forme négative d'une partie de la tige fémorale (54) du spacer de hanche à fabriquer, ladite forme creuse (12) étant limitrophe après l'insertion à l'ouverture libre restante du corps creux de l'insert adaptateur (3) ;
ensuite, une tête (21) d'un noyau (20) est insérée pour la stabilisation mécanique du spacer de hanche dans le ciment osseux à base de PMMA qui se trouve dans le corps creux de l'insert adaptateur (3) assemblé avec l'insert en demi-sphère (2), de sorte qu'une traverse (22) du noyau (20) soit agencée dans la forme creuse (12) de l'élément de base (1) ;
la forme creuse (12) de l'élément de base (1) est remplie avec du ciment osseux à base de PMMA dans la partie restante ;
ensuite, une étampe (4) avec une forme creuse d'étampe (40) en tant que forme négative de la partie restante de la tige fémorale (54) à produire du spacer de hanche est pressée sur le ciment osseux à base de PMMA dans la forme creuse (12), de sorte que la forme de tête fémorale (30), le corps creux de l'insert adaptateur (3), la forme creuse (12) de l'élément de base (1) et la forme creuse d'étampe (40) de l'étampe (4) définissent la forme du spacer de hanche à produire, le pressage de l'étampe (4) ayant pour effet que le ciment osseux à base de PMMA excédentaire soit coupé par les bords coupants (14) au niveau de la liaison entre la forme creuse (12) de l'élément de base (1) et de la forme creuse d'étampe (40) ; et
qu'ensuite, lorsque le ciment osseux à base de PMMA a durci dans le moule de fabrication de spacers, qui est formé par l'élément de base (1), l'insert en demi-sphère (2), l'insert adaptateur (3) et l'étampe (4), le spacer de hanche fini est retiré du moule de fabrication de spacers.

15. Procédé selon la revendication 14, **caractérisé en ce, que**
soit utilisé en tant que noyau (20) pour la stabilisation du spacer de hanche un noyau métallique (20), notamment un noyau en acier (20).

16. Procédé selon l'une des revendications 14 ou 15, **caractérisé en ce, que**
le procédé soit exécuté avec l'utilisation d'un moule de fabrication de spacers selon l'une des revendications 1 à 13.

17. Procédé selon l'une des revendications 14 à 16, **caractérisé en ce, que**
soit agencé au noyau (20) des écarteurs (24), de sorte qu'au moins un des écarteurs (24) soit en appui au niveau de la forme creuse (12) de l'élément de base (1), lorsque la tête (21) du noyau (20) a été insérée dans le corps creux de l'insert adaptateur (3) assemblé à l'insert en demi-sphère (2).

18. Procédé selon l'une des revendications 14 à 17, **caractérisé en ce, que** après l'insertion de l'insert adaptateur (3) avec l'insert en demi-sphère (2) dans l'évidement (10) de l'élément de base (1), l'insert adaptateur (3) soit pressé par l'élément de base (1) périphérique contre l'insert en demi-sphère (2), l'insert adaptateur (3) étant de préférence pressé contre le bord coupant (36) de l'insert en demi-sphère (2).
